# EUROPÄISCHE PATENTSCHRIFT

(11) **EP 1 063 753 B1**
(45) Veröffentlichungstag und Bekanntmachung des Hinweises auf die Patenterteilung: **22.07.2009**
(21) Anmeldenummer: 99811115.7
(22) Anmeldetag: 03.12.1999
(51) Int. Cl.: H02K 7/09

(54) **Elektrischer Drehantrieb mit einem magnetisch gelagerten Rotor**
Electric rotary drive comprising a magnetically suspended rotor
Entraînement électrique rotatif comprenant un rotor suspendu magnétiquement

(30) Priorität: 22.06.1999 EP 99810553
(43) Veröffentlichungstag der Anmeldung: 27.12.2000
(73) Patentinhaber: Levitronix LLC, Waltham, MA 02451 (US)
(72) Erfinder: Schöb, Reto, Dr., 8604 Volketswil (CH); Gempp, Thomas, 4153 Reinach (CH)
(74) Vertreter: Sulzer Management AG

(56) Entgegenhaltungen:
- EP-A- 0 588 628
- WO-A-96/31934
- WO-A-98/11650
- US-A- 5 578 880

## Beschreibung

Die Erfindung betrifft einen elektrischen Drehantrieb gemäss dem Oberbegriff des unabhängigen Anspruchs 1, sowie eine Blutpumpe mit einem solchen Drehantrieb.

Blutpumpen, die üblicherweise als Axial- oder als Zentrifugalpumpen ausgestaltet sind, dienen der Förderung von Blut und werden beispielsweise im Rahmen von Operationen am Herzen zur Aufrechterhaltung des Blutkreislaufs eingesetzt. Ferner sind implantierbare Blutpumpen bekannt, die zur temporären oder chronischen Unterstützung der Herztätigkeit in den Körper des Patienten implantiert werden.

Bei Blutpumpen muss es gewährleistet sein, dass es zu keiner Verunreinigung des geförderten Bluts kommt. Daher wird in Blutpumpen der Rotor des elektromagnetischen Antriebs und/oder der Pumpenrotor vorzugsweise magnetisch berührungslos gelagert. Diese magnetische Lagerung des Rotors kann entweder durch separate, das heisst vom Antrieb verschiedene Magnetlager realisiert werden, oder die magnetische Lagerung wird durch den Stator des Antriebs realisiert.

In der WO-A-96/31934 wird beispielsweise eine als Blutpumpe geeignete Rotationspumpe offenbart, die als sogenannter lagerloser Motor ausgestaltet ist. Dies ist ein elektromagnetischer Drehantrieb, bei welchem der Rotor mittels magnetischer Kräfte berührungslos bezüglich des Stators gelagert ist, wobei keine separaten Magnetlager für den Rotor vorhanden sind. Der Stator ist dazu als Lager- und Antriebsstator ausgestaltet, der eine Antriebswicklung und eine Steuerwicklung umfasst. Mit diesen beiden Wicklungen lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar. Bezüglich dreier weiterer Freiheitsgrade, nämlich seiner axialen Auslenkung in Richtung der Drehachse und Verkippungen bezüglich der zur Drehachse senkrechten Ebene (zwei Freiheitsgrade) ist der Rotor passiv magnetisch, das heisst nicht ansteuerbar, durch Reluktanzkräfte stabilisiert.

In diesem Sinne ist für die folgenden Ausführungen der Begriff "lagerloser Motor" zu verstehen. Bezüglich weiterer Details der Ausgestaltung und speziell der Ansteuerung bzw. Regelung des lagerlosen Motors sei hier neben der bereits zitierten WO-A-96/31934 auf die WO-95/18925 verwiesen.

Weiterhin sollen Blutpumpen, insbesondere im Falle einer Implantation in den Körper, kompakt und platzsparend sein, aber dennoch eine Pumpleistung erbringen können, die zumindest derjenigen des Herzens entspricht. Hierzu wird z. B. in der WO-A-96/31934 vorgeschlagen, den Rotor des lagerlosen Motors mit Flügeln zu versehen, sodass der Rotor des Drehantriebs identisch mit dem Pumpenrotor ist, also einen Integralrotor bildet. Dieser Rotor dient somit als Antriebsrotor, Lagerrotor und Pumpenrotor, wodurch eine sehr kompakte und leistungsfähige Blutpumpe realisierbar ist.

Ein Problem bei bekannten lagerlosen Motoren ist darin zu sehen, dass beim Auftreten von Fehlern, wie beispielsweise dem Ausfall einer Verstärkerstufe oder dem Bruch einer elektrischen Leitung in einer der Phasen der Wicklungen des Stators, ein ordnungsgemässes Funktionieren des Antriebs und/oder der magnetischen Lagerung des Rotors nicht mehr gewährleistet ist. Dies stellt insbesondere bei sehr sensiblen Anwendungen, z. B. bei implantierten Blutpumpen ein enormes Sicherheitsrisiko dar. Ein Versagen des Rotorantriebs oder der magnetischen Rotorlagerung kann nämlich sehr schlimme, eventuell sogar tödliche Folgen haben kann. Die Erfindung widmet sich daher der Aufgabe, dieses Sicherheitsrisiko deutlich zu reduzieren.

Der Erfindung liegt deshalb die Aufgabe zugrunde, einen als lagerlosen Motor ausgestalteten elektrischen Drehantrieb bereitzustellen, der sowohl bezüglich der magnetischen Lagerung des Rotors als auch bezüglich des Antriebs des Rotors fehlertolerant ist, das heisst, beim Auftreten von Fehlern soll noch ein ordnungsgemässer Betrieb des lagerlosen Motors mit zuverlässiger magnetischer Lagerung des Rotors und zuverlässigem Antrieb des Rotors möglich sein.

Der diese Aufgaben lösende elektrische Drehantrieb ist durch die Merkmale des unabhängigen Anspruchs 1 gekennzeichnet.

Der erfindungsgemässe fehlertolerante elektrische Drehantrieb, ausgestaltet als lagerloser Motor, hat also einen magnetisch gelagerten Rotor und einen Stator, der eine mindestens zwei Stränge aufweisende Antriebswicklung zum Erzeugen eines magnetischen Antriebsfelds umfasst, welches ein Drehmoment auf den Rotor bewirkt, und eine mindestens drei Stränge aufweisende Steuerwicklung zum Erzeugen eines magnetischen Steuerfelds, mit welchem die Position des Rotors bezüglich des Stators regelbar ist, wobei jeder Strang der Antriebswicklung zu einer anderen elektrischen Antriebsphase gehört, und jeder Strang der Steuerwicklung zu einer anderen elektrischen Steuerphase, sowie eine Stelleinrichtung, die jeden Strang der Antriebswicklung und jeden Strang der Steuerwicklung jeweils mit einem Phasenstrom oder einer Phasenspannung als Stellgrösse versorgt, wobei eine Kontrollvorrichtung (5) vorgesehen ist, die ein unabhängiges Ansteuern jeder einzelnen Antriebsphase und jeder einzelnen Steuerphase ermöglicht, sodass mit der Stelleinrichtung die Stellgrösse für jeden Strang der Antriebswicklung und für jeden Strang der Steuerwicklung unabhängig von den Stellgrössen für die anderen Stränge regelbar ist.

Dabei umfasst die Stelleinrichtung für jeden Strang der Antriebswicklung und für jeden Strang der Steuerwicklung einen separaten bipolaren Leistungsverstärker.

Der erfindungsgemässe, als lagerloser Motor ausgestaltete Drehantrieb arbeitet im fehlerfreien Normalbetrieb mit mindestens zwei Antriebsphasen und mindestens drei Steuerphasen. Mit "Antriebsphase" bzw. "Steuerphase" ist jeweils ein Strang der Antriebswicklung bzw. der Steuerwicklung und der ihn versorgenden Teil der Stelleinrichtung gemeint. Da die Stellgrösse, das heisst die Phasenspannung oder der Phasenstrom, für jeden Strang der Antriebswicklung und für jeden Strang der Steuerwicklung völlig unabhängig von den Stellgrössen für die anderen Stränge regelbar ist, ist jede Antriebsphase und jede Steuerphase, unabhängig von den übrigen elektrischen Phasen betreibbar. Somit kann der Drehantrieb beim Auftreten eines Fehlers in einer Antriebsphase und/oder einer Steuerphase, z. B. beim Ausfall einer kompletten Phase, mit einer reduzierten Anzahl von Antriebsphasen bzw. Steuerphasen weiter betrieben werden, ohne dass Zugeständnisse an das ordnungsgemässe Funktionieren sowohl der magnetischen Lagerung als auch des Antriebs des Rotors des Drehantriebs vonnöten sind.

Die minimale Anforderung für einen dauerhaften ordnungsgemässen Betrieb ist es, dass noch eine Antriebsphase und noch zwei Steuerphasen des Drehantriebs fehlerfrei sind, das heisst, es kann mindestens eine Antriebsphase und mindestens eine Steuerphase komplett ausfallen, ohne dass der zuverlässige Betrieb des Drehantriebs gefährdert ist. Je nachdem, mit wievielen Antriebs- und Steuerphasen der erfindungsgemässe Drehantrieb ausgestaltet ist, kann er sogar beim Ausfall mehrere Antriebs- und/oder Steuerphasen noch weiter betrieben werden.

Da der erfindungsgemässe Drehantrieb mit einer reduzierten Anzahl von Phasen betrieben werden kann, ist es grundsätzlich egal, wo in einer Antriebsphase oder in einer Steuerphase ein Fehler auftritt. So kann beispielsweise ein Leistungsverstärker ausfallen, oder es kann zum Bruch einer Leitung in einem Strang der Antriebswicklung bzw. der Steuerwicklung kommen, oder es kann ein Kurzschluss in einem Leistungsverstärker oder einem Wicklungsstrang der Antriebs- oder der Steuerwicklung auftreten, und trotz eines solchen Fehlers ist ein weiterer zuverlässiger Betrieb des Drehantriebs möglich. Aufgrund dieser hohen Fehlertoleranz bringt der erfindungsgemässe Drehantrieb eine erhebliche Steigerung der Betriebssicherheit mit sich.

Der erfindungsgemässe Drehantrieb mit seiner im fehlerfreien Normalfall mindestens zweiphasigen Ausgestaltung der Antriebswicklung ist vorzugsweise ein permanentmagnetisch erregter Drehfeldmotor, also insbesondere ein permanentmagnetisch erregter Synchronmotor oder ein bürstenloser Gleichstrommotor (letzterer ist trotz seines allgemein üblichen Namens dem Wesen nach ein Drehfeldmotor). Das heisst, das vom Stator erzeugte Antriebsfeld ist ein magnetisches Drehfeld, welches den permanentmagnetischen Rotor antreibt. Falls durch einen Fehler in einer Antriebsphase oder in mehreren Antriebsphasen nur noch eine fehlerfreie Phase für den Betrieb zur Verfügung steht, so wird der Drehfeldmotor zu einem einphasigen Wechselstrommotor.

Die Ausgestaltung des lagerlosen Motors als permanentmagnetisch erregter Drehantrieb, also mit einem permanentmagnetisch erregten Rotor, hat im Unterschied zu felderregten Drehantrieben den Vorteil, dass kein Strom und damit keine Energie für die Felderregung benötigt wird. Die permanentmagnetische Erregung ermöglicht somit einen sehr wirtschaftlichen Betrieb mit vergleichsweise geringem Energieverbrauch Dies ist insbesondere für Blutpumpen und speziell für implantierbare Blutpumpen ein wesentlicher Vorteil, weil für diese im allgemeinen kein beliebig grosser Energievorrat zu Verfügung steht.

Wie für den lagerlosen Motor üblich (siehe z. B. WO-A-98/11650) sind solche Ausgestaltungen des erfindungsgemässen Drehantriebs bevorzugt, bei denen die Antriebswicklung eine Polpaarzahl p aufweist und die Steuerwicklung eine Polpaarzahl von p+1 oder von p-1, das heisst die Polpaarzahlen der beiden Wicklungen unterscheiden sich um eins.

Gemäss einem bevorzugten Ausführungsbeispiel hat der Drehantrieb genau drei Antriebsphasen und genau drei Steuerphasen. Bei einer solchen Ausgestaltung kann der Drehantrieb auch beim kompletten Ausfall von zwei Antriebsphasen und einer Steuerphase den Rotor noch zuverlässig antreiben und lagern.

Insbesondere im Hinblick auf eine möglichst kompakte, einfache und platzsparende Ausgestaltung sind ferner solche Ausführungen bevorzugt, bei denen genau zwei Antriebsphasen und genau vier Steuerphasen vorgesehen sind. Durch diese Massnahme ist es möglich, die Anzahl der Statorzähne und die Anzahl der Spulen für die Antriebs- und die Steuerwicklung zu reduzieren, wodurch sich der apparative Aufwand, die Komplexität und die Grösse des lagerlosen Motors verringern lässt. Gleichzeitig bleibt die Fehlertoleranz gegenüber dem Ausfall einer Antriebsphase und dem Ausfall mindestens einer Steuerphase erhalten. Bei solchen Ausführungen weist der Stator vorzugsweise genau acht Statorzähne auf, zwischen denen der Rotor gelagert ist. Dies hat zusätzlich den Vorteil, dass in den Nuten zwischen den Statorzähnen mehr Platz ist, beispielsweise für Sensorelemente der Positionssensoren.

Gemäss einer bevorzugten Ausgestaltung umfasst die Steuerwicklung mehrere konzentrierte Steuerspulen, von denen jede um einen anderem Statorzahn gewickelt ist.

Im Hinblick auf eine möglichst grosse Fehlertoleranz des erfindungsgemässen lagerlosen Motors sind vorzugsweise zwei separate Kontrollsysteme vorgesehen, von denen jedes die folgenden Komponenten umfasst:
- mindestens zwei Positionssensoren zur Erfassung der radialen Position des Rotors im Stator;
- Mittel zur Bestimmung des Rotorwinkels, beispielsweise Feldsensoren;
- mindestens drei Leistungsverstärker zur Versorgung der einzelnen Stränge der Antriebs- und der Steuerwicklung
- eine Signalverarbeitungs- und Regeleinrichtung zur Regelung des Antriebs und der Position des Rotors sowie zur Ansteuerung der Leistungsverstärker;
wobei jedes Kontrollsystem mindestens eine Antriebsphase und mindestens zwei Steuerphasen ansteuert.

Durch diese Massnahme wird der lagerlose Motor durch zwei fast identische Kontrollsysteme betrieben, die unabhängig voneinander arbeiten können. Da jedes Kontrollsystem die für den Betrieb des lagerlosen Motors notwendigen Komponenten, wie z. B. Positionssensoren, Mittel zur Bestimmung des Rotorwinkels sowie Regeleinrichtungen und Leistungsverstärker für mindestens eine Antriebsphase und mindestens zwei Steuerphasen umfasst, kann jedes Kontrollsystem alleine und unmittelbar die Ansteuerung und die Regelung des lagerlosen Motors übernehmen. Im fehlerfreien Normalbetrieb steuern beide Kontrollsysteme gemeinsam den lagerlosen Motor. Im Falle eines Fehlers kann jedoch der zuverlässige Betrieb sofort mit einem der beiden Kontrollsysteme gewährleistet werden. Diese sogenannte heisse Redundanz bringt noch eine weitere erhebliche Steigerung der Fehlertoleranz und damit der Betriebssicherheit mit sich.

Vorzugsweise sind ferner Kommunikationsmittel zur Kommunikation zwischen den beiden Kontrollsystemen vorgesehen. Hieraus resultiert insbesondere im Normalbetrieb, in dem beide Kontrollsysteme aktiv sind, eine weitere Erhöhung der Sicherheit. Da beispielsweise jedes Kontrollsystem über eigene Positionssensoren verfügt, ist die Ermittlung der Position des Rotors im Normalbetrieb überbestimmt. Indem die Kontrollsysteme über die Kommunikationsmittel die von ihnen jeweils ermittelten Werte für die Position des Rotors austauschen, kann diese Redundanz vorteilhaft zur Überprüfung der Positionssensoren genutzt werden. Analoges gilt für die Feldsensoren bzw. den Rotorwinkel.

Ferner sind bevorzugt Fehlererkennungsmittel vorgesehen, mit denen Fehler in den Kontrollsystemen und/oder in den einzelnen Antriebsphasen und oder in den einzelnen Steuerphasen und/oder in der Energieversorgung detektierbar sind. Diese Fehlererkennungsmittel können als Komponenten sowohl der Hardware als auch der Software ausgestaltet sein. Gemäss einer weiteren vorteilhaften Massnahme können die Fehlererkennungsmittel im Falle der Detektion eines Fehlers in Abhängigkeit von der Art des Fehlers diesen beheben oder den Drehantrieb in einen geeigneten von mehreren möglichen Fehlermodi umschalten. In diesen Fehlermodi können Teile eines Kontrollsystems oder auch ein gesamtes Kontrollsystem, oder einzelne Stränge der Antriebs- oder Steuerwicklung deaktiviert werden. Dadurch, dass mehr als ein Fehlermodus vorgesehen ist, können die Kontrollsysteme sehr flexibel und an den jeweils aufgetretenen Fehler angepasst reagieren und nur die jeweils notwendigen Komponenten ausser Betrieb setzen bzw. nicht mehr verwenden.

Insbesondere im Hinblick auf Blutpumpen soll der erfindungsgemässe lagerlose Motor sehr kompakt und platzsparend ausgestaltet werden können. Diesbezüglich ist es vorteilhaft, jedes Kontrollsystem zumindest teilweise in Form von Elektronikprints auszugestalten, die im Innern des Drehantriebs angeordnet sind.

Vorzugsweise umfassen dabei die im Innem des Drehantriebs angeordneten Elektronikprints alle Leistungsverstärker und sind so angeordnet, dass die einzelnen Leistungsverstärker jeweils unmittelbar mit den zugehörigen Antriebs- bzw. Steuerspulen verbunden sind, also ohne Kabel. Durch diese Massnahme lässt sich die Anzahl der vom lagerlosen Motor nach aussen weggeführten Kabeladern auf ein Minimum reduzieren. Da erfahrungsgemäss Kabelverbindungen die höchste Ausfallrate aller Komponenten haben, resultiert hieraus eine weitere Steigerung der Sicherheit.

Das Anordnen der Elektronikprints im Innem des Drehantriebs ist insbesondere in Kombination mit einer bevorzugten Ausgestaltung des Drehantriebs als sogenannter Tempelmotor problemlos möglich. Bei einem Tempelmotor, wie er beispielsweise in der WO-A-98/11650 offenbart ist (siehe dort Fig. 8k und zugehörige Textpassagen) weist der Stator mehrere durch einen Rückschluss verbundene Statorzähne auf, die jeweils L-förmig ausgebildet sind, wobei sich der längere Schenkel in der axialen Richtung erstreckt, welche durch die Soll-Drehachse des Rotors festgelegt ist, und der kürzere Schenkel radial nach innen verläuft. Hier können in dem von den längeren Schenkeln der Statorzähne umgebenen Raum Elektronikprints angeordnet werden.

Eine weitere vorteilhafte Massnahme besteht darin, bei einem Tempelmotor ein Auswertemodul für die Positionssensoren vorzusehen, welches als Elektronikprint ausgestaltet ist und derart auf den kürzeren Schenkeln der Statorzähne angeordnet ist, dass sich die auf dem Elektronikprint vorgesehenen Bauteile in dem freien Raum zwischen den Statorzähnen befinden. Hieraus resultiert eine optimale Raumausnutzung im lagerlosen Motor.

Auch ist es vorteilhaft, einzelne Elektronikprints durch flexible Verbindungsprints (Flexprints) miteinander zu verbinden. Somit bilden nämlich die im wesentlichen festen Elektronikprints und die flexiblen Verbindungsprints einen Verbund, einen sogenannten Rigid-Flex-Verbund, welcher als Einheit gefertigt, bestückt und geprüft werden kann.

Durch die Erfindung wird ferner eine Blutpumpe mit einem erfindungsgemässen elektrischen Drehantrieb vorgeschlagen, wobei der Rotor des Drehantriebs permanentmagnetisch erregt ist und mehrere Flügel zum Fördern des Bluts aufweist, sodass der Rotor des Drehantriebs auch als Pumpenrotor dient. Eine derartige Blutpumpe ist in hohem Masse fehlertolerant, extrem sicher, kompakt, leistungsfähig und sparsam hinsichtlich des Energiebedarfs. Sie eignet sich für Anwendungen innerhalb und ausserhalb des Körpers.

Weitere vorteilhafte Massnahmen und bevorzugte Ausgestaltungen der Erfindung ergeben sich aus den abhängigen Ansprüchen.

Im Folgenden wird die Erfindung anhand von Ausführungsbeispielen und anhand der Zeichnung näher erläutert. In der schematischen Zeichnung, zeigen:
- Fig. 1:: eine schematische Darstellung eines ersten Ausführungsbeispiels des erfindungsgemässen Drehantriebs,
- Fig. 2:: den Stator und den Rotor des ersten Ausführungsbeispiels in einer Aufsicht (Rotor geschnitten),
- Fig. 3:: einen Schnitt durch den Stator aus Fig. 2 entlang der Schnittlinie III-III in Fig. 2
- Fig. 4:: eine schematische Darstellung der Anordnung und der Verschaltung der Steuerspulen,
- Fig. 5:: eine schematische Darstellung der Anordnung und der Verschaltung der Antriebsspulen,
- Fig. 6:: ein Ausführungsbeispiel eines bipolaren Leistungsverstärkers,
- Fig. 7:: den Stator einer Variante des ersten Ausführungsbeispiels in einer Aufsicht,
- Fig. 8:: wie Fig. 4, jedoch für die Variante gemäss Fig. 7,
- Fig. 9:: eine perspektivische Darstellung eines zweiten Ausführungsbeispiels des erfindungsgemässen Drehantriebs, ausgestaltet als Tempelmotor, teilweise im Schnitt,
- Fig. 10:: analog zu Fig. 2, jedoch für ein drittes Ausführungsbeispiel des Drehantriebs,
- Fig. 11:: eine Blockdarstellung eines Ausführungsbeispiels einer Kontrollvorrichtung,
- Fig. 12:: ein Blockschaltbild einer Signalverarbeitungs- und Regeleinrichtung der Kontrollvorrichtung gemäss Fig.11.
- Fig. 13:: ein Blockdiagramm einer FPGA-Logik,
- Fig. 14:: eine Blockdarstellung von Interprozessorverbindungen,
- Fig. 15:: eine Blockdarstellung eines Leistungsteils,
- Fig. 16:: eine Blockdarstellung eines Zwei-Achsen-Messsystems,
- Fig. 17:: eine Blockdarstellung eines Feldsensors,
- Fig. 18:: eine Blockdarstellung eines Versorgungskreises,
- Fig. 19:: ein Flussdiagramm eines Software-Zyklus,
- Fig. 20a-c:: ein Flussdiagramm einer Fehlererkennungsroutine, und
- Fig. 21:: eine schematische Schnittdarstellung eines Ausführungsbeispiels einer erfindungsgemässen Blutpumpe.

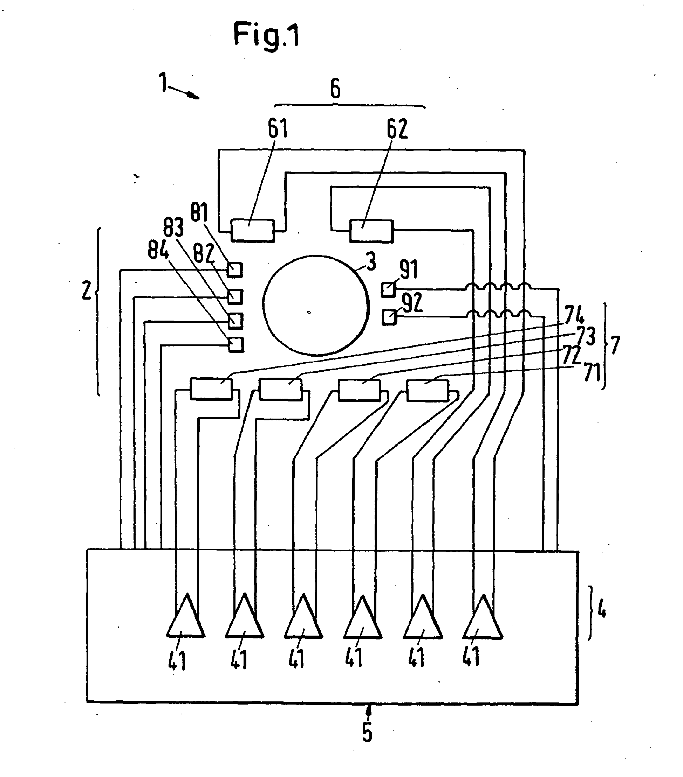

Fig. 1 zeigt in einer schematischen Blockdarstellung die wesentlichen Teile eines ersten Ausführungsbeispiels des erfindungsgemässen Drehantriebs, der als lagerloser Motor ausgestaltet ist und gesamthaft mit dem Bezugszeichen 1 versehen ist. Der Drehantrieb 1 umfasst einen Stator 2, einen permanentmagnetischen Rotor 3 sowie eine Kontrollvorrichtung 5 mit einer Stelleinrichtung 4, die mehrere bipolare Leistungsverstärker 41 umfasst. Die Kontrolleinrichtung 5 umfasst ferner die gesamten Ansteuer- und Regeleinheiten, die für den Betrieb des Drehantriebs 1 notwendig sind. Diese sind in Fig. 1 aus Gründen der besseren Übersicht nicht näher dargestellt. Der Aufbau der Kontrollvorrichtung 5 wird weiter hinten noch detailliert beschrieben.

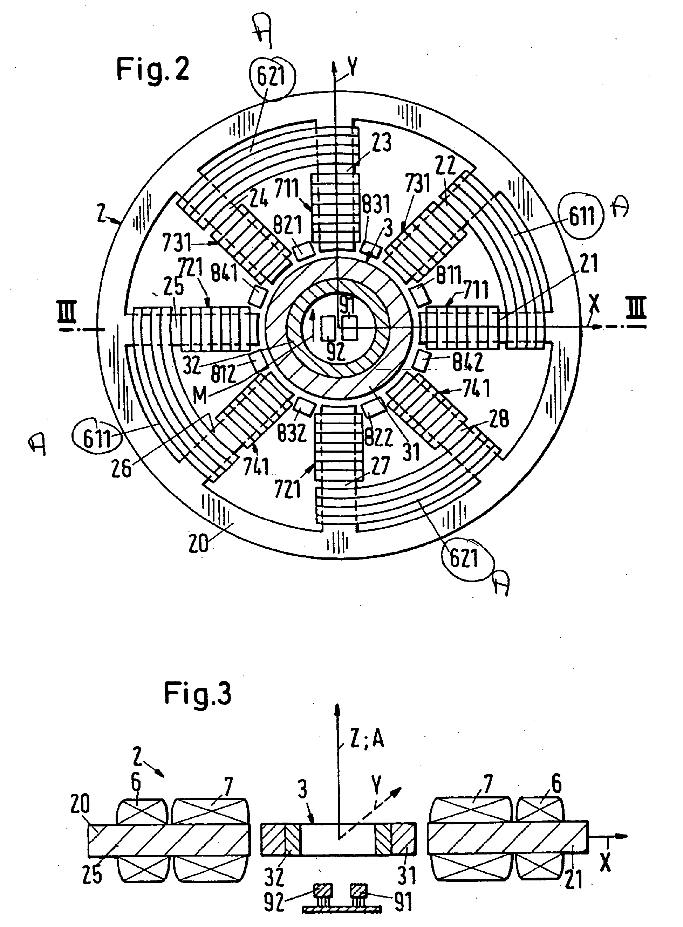

Der Stator 2 und der Rotor 3 sind in Fig. 2 in einer Aufsicht dargestellt und in Fig. 3 in einem Schnitt entlang der Linie III-III in Fig. 2.

Fig. 3 veranschaulicht zudem die Festlegung eines Statorsystems, auf das in der Beschreibung Bezug genommen wird. Das Statorsystem ist ein kartesisches Koordinatensystem mit den Achsen X,Y und Z, dessen Ursprung im Zentrum des Stators liegt und das bezüglich des Stators 2 ortsfest ist. Konventionsgemäss zeigt die Z-Achse in Richtung der Soll-Drehachse des Rotors 3, womit diejenige Drehachse A gemeint ist, um die der Rotor 3 im Betriebszustand rotiert, wenn er sich in einer exakt zentrierten Position (Soll-Position) bezüglich des Stators 2 befindet, also insbesondere auch nicht verkippt ist. Die Richtung der Z-Achse wird im folgenden als "axiale Richtung" bezeichnet. Die Richtung der X- und der Y-Achse des Statorsystems ist willkürlich. Die durch die X- und die Y-Achse aufgespannte X-Y-Ebene ist die Ebene, in welcher der Rotor 3 rotiert, falls er nicht verkippt oder in axialer Richtung ausgelenkt ist. Die Position des Rotors 3 (bzw. seines Zentrums) bezüglich der X-Y-Ebene wird im Folgenden als seine "radiale Position" bezeichnet.

Gemäss dem Prinzip des lagerlosen Motors umfasst der Stator 2 eine Antriebswicklung 6 zum Erzeugen eines magnetischen Antriebsfelds, welches ein Drehmoment auf den Rotor 3 bewirkt und diesen antreibt, sowie eine Steuerwicklung 7 zum Erzeugen eines magnetischen Steuerfelds, mit welchem die radiale Position des Rotors 3 regelbar ist. Durch die Kombination dieser beiden magnetischen Drehfelder kann der Rotor 3 angetrieben und magnetisch berührungslos im Stator 2 gelagert werden, ohne dass dafür separate Magnetlager notwendig sind. Bei dem lagerlosen Motor werden drei Freiheitsgrade des Rotors, nämlich seine Rotation um die Drehachse A und seine radiale Position (zwei Freiheitgrade) aktiv magnetisch gesteuert bzw. geregelt. Bezüglich dreier weiterer Freiheitsgrade, nämlich seiner Auslenkung in axialer Richtung und Verkippungen relativ zur Z-Achse (zwei Freiheitsgrade) ist der Rotor passiv magnetisch, das heisst nicht ansteuerbar, durch Reluktanzkräfte stabilisiert. Weitere Details bezüglich der Funktionsweise des lagerlosen Motors sowie seiner Regelung und Ansteuerung sind in den bereits zitierten Druckschriften WO-A-96/31934, WO-A-95/18925 und WO-A-98/11650 offenbart und werden deshalb hier nicht weiter erläutert.

Bei dem ersten Ausführungsbeispiel hat die Antriebswicklung 6 zwei Stränge 61,62, die jeweils zu einer anderen Antriebsphase gehören, das heisst die Antriebswicklung 6 ist zweiphasig (mit zwei Antriebsphasen) ausgestaltet. Die Steuerwicklung 7 hat vier Stränge 71,72,73,74, die jeweils zu einer anderen Steuerphase gehören, das heisst die Steuerwicklung ist vierphasig (mit vier Steuerphasen) ausgestaltet.

Der Stator 2 umfasst mehrere, beim ersten Ausführungsbeispiel acht, Statorzähne 21-28, die mittels eines Rückschlusses 20, üblicherweise ein Eisenrückschluss, magnetisch aneinander gekoppelt sind. Um die Statorzähne 21-28 sind die Antriebs- und die Steuerwicklung 6 bzw. 7 in Form von diskreten Spulen gewickelt. Dabei können mehrere diskrete Spulen elektrisch in einer Parallel- oder in einer Serienschaltung miteinander verbunden sein. Mit der Bezeichnung "Strang" ist jeweils die Gesamtheit aller der diskreten Spulen gemeint, die elektrisch parallel oder seriell zusammengeschaltet sind. Natürlich kann ein Strang auch nur eine diskrete Spule umfassen. Mit dem Begriff "Phase" wird jeweils ein Strang und der ihn versorgende Teif der Stelleinrichtung 4, also hier insbesondere der Leistungsverstärker 41, der diesen Strang mit einem Phasenstrom oder mit einer Phasenspannung als Stellgrösse versorgt, bezeichnet. Die Stelleinrichtung 4 kann als Stromsteller oder als Spannungssteller für die Antriebs- und die Steuerwicklung 6,7 ausgebildet sein. Da in der Praxis meistens Stromregelungen Verwendung finden, wird im Folgenden auf den Fall Bezug genommen, dass die Stellgrössen Phasenströme sind. Für Spannungsregelungen mit Phasenspannungen als Stellgrösse gelten jedoch sinngemäss die gleichen Erläuterungen.

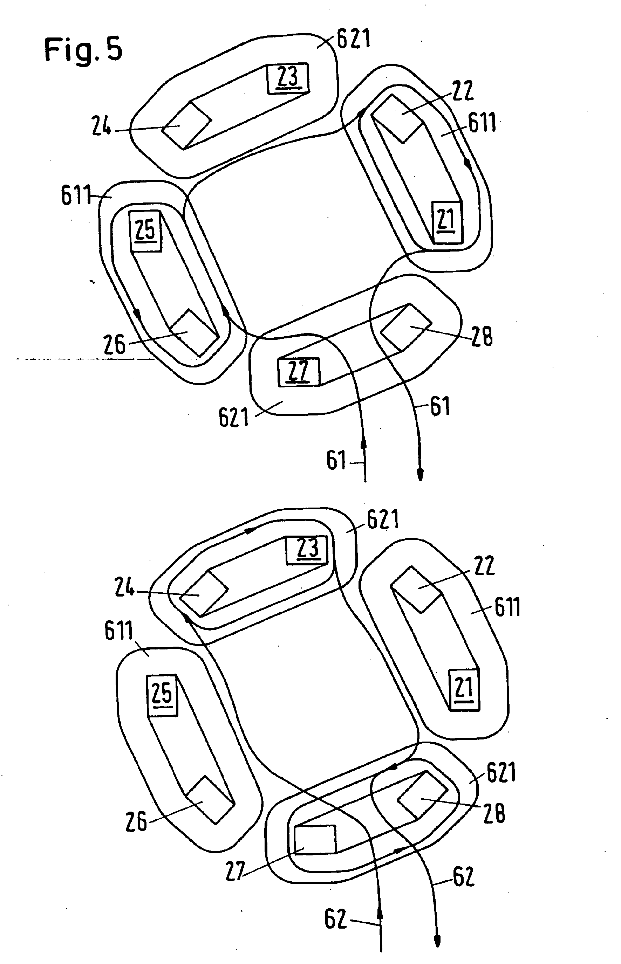

Wie dies Fig. 2 zeigt besteht die zweiphasige Antriebswicklung 6 beim ersten Ausführungsbeispiel aus vier konzentrierten Spulen, die im Folgenden als Antriebsspulen 611, 621 bezeichnet werden. Die beiden Antriebsspulen 611 bilden den einen Strang 61 der Antriebswicklung 6, während die beiden Antriebsspulen 621 den anderen Strang 62 der Antriebswicklung 6 bilden. Antriebsspulen, die zur gleichen Antriebsphase gehören sind mit identischen Bezugszeichen bezeichnet. Jede Antriebsspule 611, 621 ist als konzentrierte Spule jeweils um zwei unmittelbar benachbarte Statorzähne 21-28 gewickelt, derart, dass keiner der Statorzähne 21-28 mehr als eine Antriebsspule 611, 621 trägt. Das heisst, die erste Antriebsspule 611 ist um den ersten und zweiten Statorzahn 21 bzw. 22 gewickelt, die nächste Antriebsspule 621 um den dritten und vierten Statorzahn 23 bzw. 24, die nächste Antriebsspule 611 um den fünften und sechsten Statorzahn 25 bzw. 26 und die letzte Antriebsspule 621 um den siebten und achten Statorzahn 27 und 28. Die Antriebsspulen 611,621 sind so zusammengeschaltet, dass jeweils zwei, sich diametral gegenüberliegende Antriebsspulen 611 oder 621 einen Strang 61 oder 62 der Antriebswicklung 6 bilden und somit zur gleichen Antriebsphase gehören. Die Verteilung der Antriebsspulen 611,621 auf die Statorzähne 21-28 und ihre Verschaltung zu den beiden Strängen 61,62 der Antriebswicklung ist in Fig. 5 nochmals in einer mehr schematischen Darstellung verdeutlicht, wobei im oberen Teil der Fig. 5 der Strang 61 der ersten Antriebsphase und im unteren Teil der Fig. 5 der Strang 62 der zweiten Antriebsphase veranschaulicht ist.

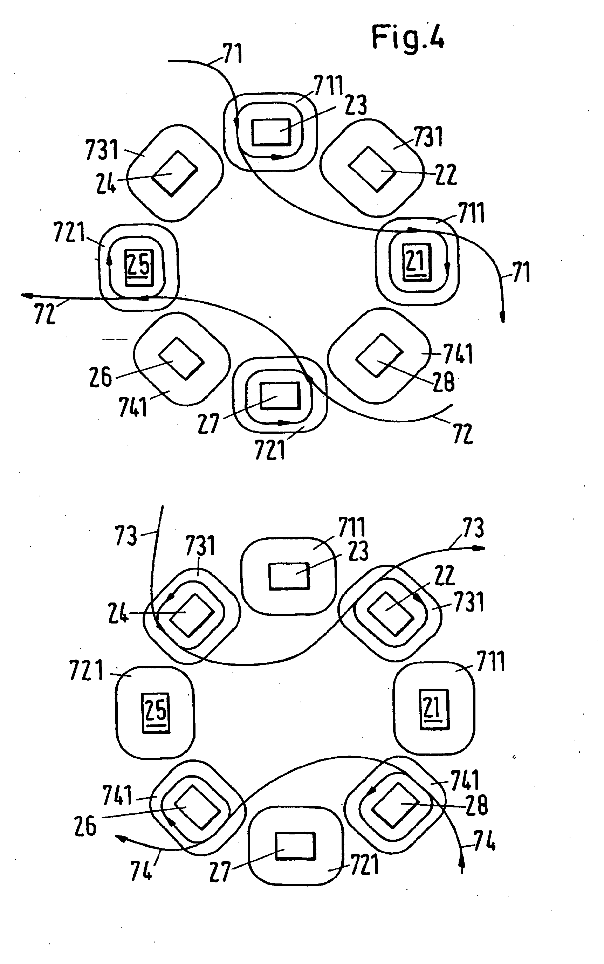

Fig. 2 zeigt ebenfalls die vierphasige Steuerwicklung 7, die acht konzentrierte Spulen umfasst, welche im Folgenden als Steuerspulen 711,721,731,741 bezeichnet werden. Jede Steuerspule 711,721,731,741 ist um einen anderen Statorzahn 21-28 gewickelt, das heisst jeder Statorzahn trägt genau eine Steuerspule. Jeweils zwei Steuerspulen 711 oder 721 oder 731 oder 741 bilden einen Strang 71 oder 72 oder 73 oder 74 der Steuerwicklung 7 und gehören somit zur gleichen Steuerphase. Die Steuerspulen, die zur gleichen Steuerphase gehören, sind mit identischen Bezugszeichen bezeichnet. Die Steuerspulen sind so zusammengeschaltet, dass jeweils die um einen ersten Statorzahn gewickelte Steuerspule und die um den in Umfangsrichtung des Stators gesehen übernächsten Statorzahn gewickelte Steuerspule zur gleichen Steuerphase gehören. Diese Verschaltung der einzelnen Steuerspulen 711,721,731,741 ist in Fig. 4 in einer schematischen Darstellung verdeutlicht. Die Steuerspule 711, die um den ersten Statorzahn 21 gewickelt ist (Fig. 4, oben) und die Steuerspule 711, die um den dritten Statorzahn 23 gewickelt ist, sind zusammengeschaltet und bilden den Strang 71 der ersten Steuerphase der Steuerwicklung 7. Die beiden Steuerspulen 721 auf dem fünften bzw. siebten Statorzahn 25 bzw. 27 bilden den Strang 72 der zweiten Steuerphase. Die beiden Steuerspulen 731 auf dem zweiten bzw. vierten Statorzahn 22 bzw. 24 bilden den Strang 73 der dritten Steuerphase (Fig. 4, unten), und die beiden Steuerspulen 741 auf dem sechsten bzw. achten Statorzahn 26 bzw. 28 bilden den Strang 74 der vierten Steuerphase.

Mit dieser Ausgestaltung der Antriebs- und Steuerphasen hat die Antriebswicklung eine Polpaarzahl eins und die Steuerwicklung eine Polpaarzahl zwei. Allgemein werden für den erfindungsgemässen Drehantrieb 1 solche Ausgestaltungen bevorzugt, bei denen sich die Polpaarzahlen der Antriebswicklung und der Steuerwicklung um eins unterscheiden, das heisst, wenn p die Polpaarzahl der Antriebswicklung bezeichnet, dann hat die Steuerwicklung vorzugsweise die Polpaarzahl p±1 also entweder p+1 oder p-1. Aus praktischen Gründen sind solche Ausgestaltungen besonders bevorzugt, bei denen die Polpaarzahl der Antriebswicklung p=1 oder p=2 oder p=3 ist und die Polpaarzahl der Steuerwicklung eins oder zwei oder drei beträgt.

Erfindungsgemäss ist die Stelleinrichtung 4 (Fig. 1) so ausgestaltet, dass der Phasenstrom (oder die Phasenspannung) als Stellgrösse für jeden Strang 61,62 der Antriebswicklung und für jeden Strang 71-74 der Steuerwicklung unabhängig von den Phasenströmen (oder Phasenspannungen) für die anderen Stränge regelbar ist. Dazu umfasst die Stelleinrichtung beim ersten Ausführungsbeispiel sechs separate bipolare Leistungsverstärker 41, nämlich für jede der beiden Antriebsphasen und für jede der vier Steuerphasen jeweils einen. Hierdurch ist gewährleistet, dass auch beim Ausfall einer kompletten Antriebs- und/oder Steuerphase die noch verbleibenden fehlerfreien Phasen uneingeschränkt weiter betrieben werden können.

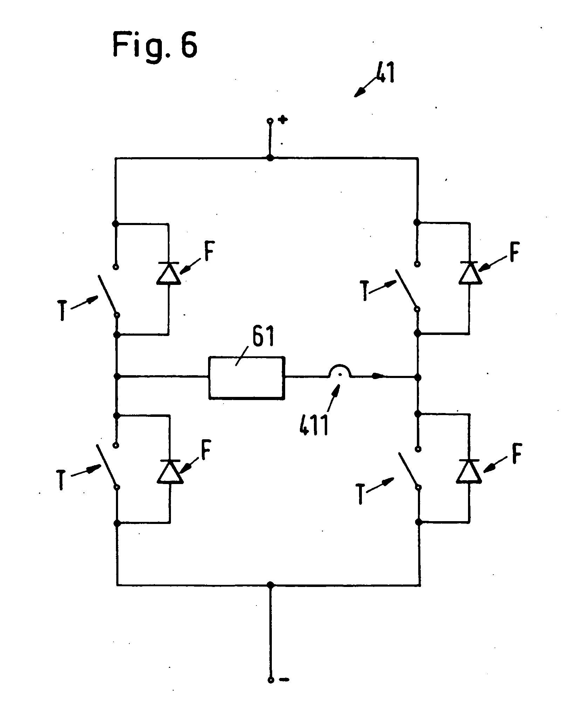

In einer bevorzugten Ausgestaltung sind die bipolaren Leistungsverstärker 41 jeweils H-Brücken Schaltverstärker. Fig. 6 zeigt den elektrischen Schaltplan eines solchen Leistungsverstärkers 41, der eine der Phasen (hier den Strang 61 der Antriebswicklung) mit dem Phasenstrom versorgt. Der Leistungsverstärker 41 ist in an sich bekannter Weise mit den Schalttransistoren T und Freilaufdioden F ausgestaltet und wird mit den beiden Betriebspotentioalen + und - betrieben, wobei das Betriebspotential - beispielsweise das Erdpotential GND ist und das Betriebspotential + ein Potential von +12V ist. Die Schalttransistoren T sind vorzugsweise Feldeffekttransistoren (FET) und insbesondere MOS-FETs. Zum Messen des jeweiligen Phasenstroms ist ein Strommessgerät 411 vorgesehen.

Natürlich können bei dem erfindungsgemässen Drehantrieb 1 auch andere Typen von Leistungsverstärkern 41 bzw. Stelleinrichtungen 4 vorgesehen sein. Beispielsweise können die einzelnen Leistungsverstärker auch als Brückenzweige eines mehrphasigen Stromstellers realisiert sein und mehrere Phasen in einer Sternpunktschaltung zusammengeschaltet sein, wobei dann jedoch der Sternpunkt auf einem belastbaren Potential liegen muss, um die unabhängige Regelung der einzelnen Phasenströme zu gewährleisten. Eine solche belastbare Sternpunktschaltung ist beispielsweise in der europäischen Patentanmeldung Nr. 99810553.0 der gleichen Anmelderin offenbart und wird daher hier nicht näher erläutert. Es können aber auch andere Formen von Schaltverstärkern oder Analogverstärker verwendet werden. Wichtig ist jedoch, dass der Leistungsverstärker 41 so ausgebildet ist, dass er bipolar betreibbar ist, womit gemeint ist, dass sowohl die Phasenströme als auch die Phasenspannungen positives und negatives Vorzeichen annehmen können. Zusätzlich muss die Stelleinrichtung 4 jede Antriebs- und jede Steuerphase mit jeweils einer Stellgrösse (Phasenstrom bzw. Phasenspannung) versorgen können, die unabhängig von den Stellgrössen für die anderen Phasen regelbar ist.

Der permanentmagnetische Rotor 3 des Drehantriebs 1 (siehe Fig. 2 und Fig. 3) ist zweipolig (Polpaarzahl eins) diametral magnetisiert und als ringförmiger Rotor 3 ausgestaltet mit einem permanentmagnetischen Ring 31 und einem Eisenrückschluss 32, der radial innenliegend bezüglich des Rings 31 angeordnet ist. Die Magnetisierung des Rotors ist in Fig. 2 symbolisch durch den Pfeil M dargestellt. Natürlich sind auch andere Polpaarzahlen und andere Formen der Rotormagnetisierung möglich. Der Rotor 3 kann insbesondere auch scheibenförmig ausgebildet sein.

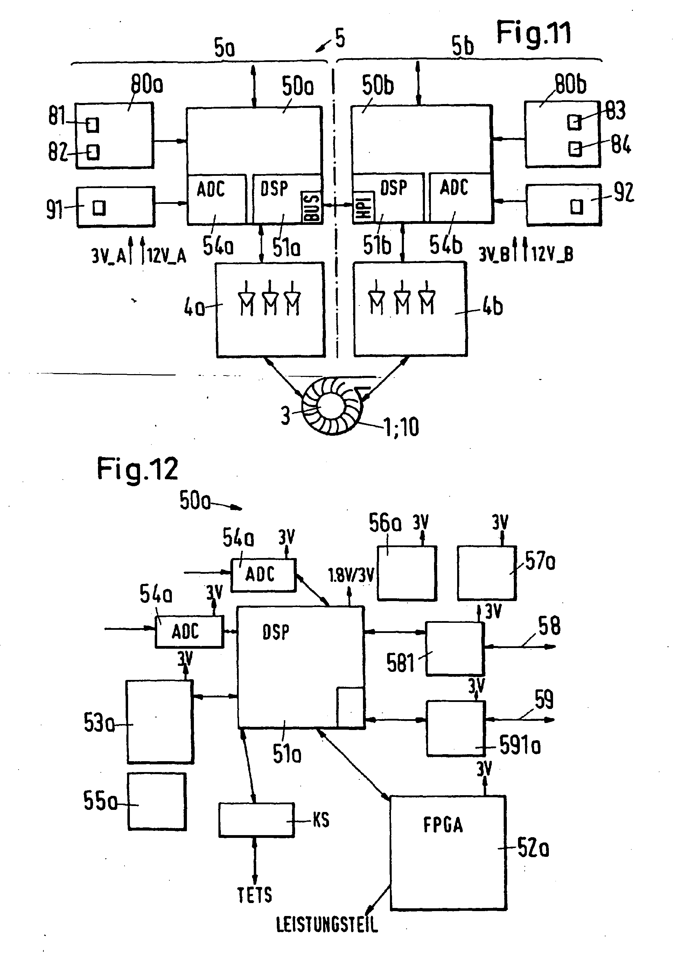

Zur Erfassung der radialen Position des Rotors 3 im Stator 2 sind mindestens zwei, bei diesem Ausführungsbeispiel vier Positionssensoren 81,82,83,84 (siehe Fig. 1) vorgesehen, von denen jeweils zwei, nämlich die Positionssensoren 81,82 bzw. die Positionssensoren 83,84 zu einem Zwei-Achsen-Messsystem 80a, 80b (siehe Fig. 11) gehören, mit welchem die radiale Position des Rotors 3 bestimmbar ist. Da somit zwei unabhängige Messsysteme für die radiale Position des Rotors vorliegen, ist die Positionsmessung des Rotors 3 redundant ausgelegt, wodurch sich die Fehlertoleranz des Drehantriebs 1 erhöht. Dies wird weiter hinten noch erläutert.

Vorzugsweise umfasst jeder Positionssensor 81-84 zwei Sensorelemente 811,812; bzw. 821,822 bzw. 831,832 bzw. 841,842 (siehe Fig. 2). Jedes Sensorelement ist in einer Nute zwischen zwei benachbarten Statorzähnen 21-28 dem Rotor 3 gegenüberliegend angeordnet, sodass sich in jeder der acht Nuten genau ein Sensorelement befindet. Die Sensorelemente können entweder in der X-Y-Ebene oder leicht axial versetzt zu dieser angeordnet sein. Jeweils zwei Sensorelement 811,812; bzw. 821,822 bzw. 831,832 bzw. 841,842, die zum gleichen Positionssensor 81 bzw. 82 bzw. 83 bzw. 84 gehören, sind bezüglich der Umfangsrichtung des Stators 2 um 180° versetzt zueinander angeordnet, sodass sie sich diametral im Stator 2 gegenüberliegen (siehe Fig. 2). Durch eine solche Anordnung können systematische Fehler, wie Common-Mode Störungen, Offsets, Driften, insbesondere thermische Driften in den Positionssensoren 81-84 eliminiert werden, indem jeweils das Differenzsignal aus den beiden Signalen der Sensorelemente, die zum gleichen Positionssensor 81-84 gehören, für die Bestimmung der Rotorposition herangezogen wird.

Die beiden Positionssensoren, die zum gleichen Zwei-Achsen-Messsystem gehören, also z. B. die beiden Positionssensoren 81 und 82 mit ihren vier Sensorelementen 811,812,821,822, sind relativ zueinander um etwa 90° bezüglich der Umfangsrichtung des Stators versetzt angeordnet und relativ zu den Positionssensoren 83,84 des anderen Zwei-Achsen-Messsystems um etwa 45° versetzt.

Als Sensorelemente 811-842 sind alle an sich bekannten Typen von Distanzsensoren geeignet, insbesondere Wirbelstromsensoren, induktive Sensoren, Magnetfeldsonden wie Hall-Sensoren, optische Sensoren, kapazitive Sensoren. Bevorzugt sind die Sensorelemente Wirbelstromsensoren.

Für die Ansteuerung und Regelung des lagerlosen Motors (Antrieb und Lage des Rotors 3), die üblicherweise mittels eines Vektorregelungs- bzw. eines feldorientierten Regelungsverfahrens erfolgt, ist es notwendig, die Richtung der Rotormagnetisierung, das heisst die momentane Lage der Magnetisierung des Rotors 3 relativ zur X-Y-Ebene des ortsfesten Statorsystems zu kennen. Diese Grösse wird üblicherweise als Rotorfeldwinkel bezeichnet. In der Praxis unterscheidet sich dieser nur geringfügig vom geometrischen Rotorwinkel. Im Folgenden wird daher dieser Unterschied nicht mehr berücksichtigt und kurz vom Rotorwinkel gesprochen,
wobei dieser Begriff sowohl den geometrischen Rotorwinkel als auch den Rotorfeldwinkel umfasst. Zur Bestimmung des Rotorwinkels ist mindestens ein Feldsensor vorgesehen. Um den Drehantrieb 1 auch bezüglich der Bestimmung des Rotorwinkels redundant auszugestalten, sind zwei unabhängige Feldsensoren 91, 92 vorgesehen. Jeder Feldsensor 91,92 umfasst zwei magnetoresistive Feldmessbrücken, wobei die magnetoresistiven Elemente beispielsweise Giant-Magneto-Resistive- (GMR) Elemente sind. Jeder der Feldsensoren 91,92 liefert z. B. jeweils den Sinus und den Cosinus des von ihm gespürten Feldes, woraus der Rotorwinkel bestimmbar ist. Somit sind zwei unabhängige Systeme vorhanden, mit denen jeweils der Rotorwinkel bestimmbar ist. Wie dies insbesondere Fig. 3 zeigt, sind die Feldsensoren 91 und 92 axial versetzt zur X-Y-Ebene, nämlich darstellungsgemäss unterhalb des Rotors 3 angeordnet. Bezüglich weiterer Details der Bestimmung des Rotorwinkels mittels derart angeordneter Feldsensoren 91,92 wird auf die Europäische Patentanmeldung Nr. 99810634.8 der gleichen Anmelderin verwiesen, welche eine Sensoranordnung zur Bestimmung der Richtung der Rotormagnetisierung zum Gegenstand hat.

Es versteht sich, dass anstelle oder in Ergänzung der hier erläuterten Feldsensoren 91,92 auch andere Mittel zur Bestimmung des Rotorwinkels vorgesehen sein können.

Im normalen Betriebszustand des Drehantriebs 1 wird mittels der zweiphasigen Antriebswicklung 6 das magnetische Antriebsfeld erzeugt, welches ein Drehmoment auf den Rotor 3 erzeugt und diesen in Rotation versetzt. Mittels der vierphasigen Steuerwicklung 7 wird die radiale Position des Rotors 3 geregelt, die mit Hilfe der Positionssensoren 81-84 bestimmbar ist. Für die Positionsregelung des Rotors 3 sind aus der Literatur zahlreiche Ausgestaltungen bekannt. Speziell für den lagerlosen Motor werden z. B. in der WO-A-95/18925 einige Regelstrukturen beschrieben. Daher wird hier auf die Ausgestaltung der Regelung des lagerlosen Motors nicht mehr eingegangen.

Kommt es nun während des Betriebs zum Ausfall einer Steuerphase und/oder zum Ausfall einer Antriebsphase, so kann der erfindungsgemässe Drehantrieb 1 mit einer reduzierten Anzahl von Antriebsphasen und/oder Steuerphasen ordnungsgemäss weiterbetrieben werden, weil der Phasenstrom für jede einzelne Antriebsphase und für jede einzelne Steuerphase vollkommen unabhängig von den anderen Phasenströmen regelbar ist. Da es die Minimalanforderung für einen zuverlässigen Antrieb und für eine zuverlässige magnetische Lagerung des Rotors ist, dass mindestens eine Antriebsphase und mindestens zwei Steuerphasen fehlerfrei arbeiten, können bei dem ersten Ausführungsbeispiel im Extremfall eine Antriebsphase und zwei Steuerphasen komplett ausfallen bzw. deaktiviert werden, ohne dass der Drehantrieb 1 seine Funktionstüchtigkeit verliert. Auf die Behandlung der Fehler wird weiter hinten noch näher eingegangen. Bezüglich weiterer Einzelheiten der Fehlertoleranz hinsichtlich der Lagerfunktion einerseits und der Antriebsfunktion andererseits sei hier auf die beiden europäischen Patentanmeldungen Nr. 99810760.1 und Nr. 99810553.0 der gleichen Anmelderin verwiesen, deren Offenbarungen hiermit zum integralen Bestandteil dieser Anmeldung erklärt werden.

Falls nur noch eine Antriebsphase fehlerfrei ist, so arbeitet der Drehantrieb 1 nach dem Prinzip des einphasigen Wechselstrommotors. Solange der Drehantrieb 1 in diesem Falle nicht angehalten wird, dreht er weiter, arbeitet also noch korrekt. Falls ein solcher einphasiger Wechselstrommotor jedoch angehalten wird, ist es je nach relativer Stellung des Rotors 3 zum Stator 2 möglich, dass der Drehantrieb 1 nicht mehr ohne weiteres angefahren werden kann. Es ist dann jedoch möglich, durch ein entsprechendes Aktivieren der noch fehlerfreien Steuerphasen den Rotor 3 entlang des Stators 2 abzurollen, solange, bis sich der Rotor 3 in einer Stellung befindet, in der er mit einer Antriebsphase angefahren werden kann. Sobald der Rotor 3 wieder dreht, kann dann der Betrieb mit nur einer Antriebsphase und mindestens zwei Steuerphasen aufrechterhalten werden.

Dem Problem des Anfahrens mit nur einer Antriebsphase kann auch abgeholfen werden, indem der Stator 2 so ausgestaltet wird, dass der Rotor 3 beim Anhalten in einer vorgegebenen "Raststellung" stehenbleibt, die so gewält wird, dass der Rotor 3 aus dieser Raststellung wieder anlaufen kann. Diese Massnahme ist von einphasigen Wechselstrommotoren hinreichend bekannt und wird daher hier nicht näher erläutert.

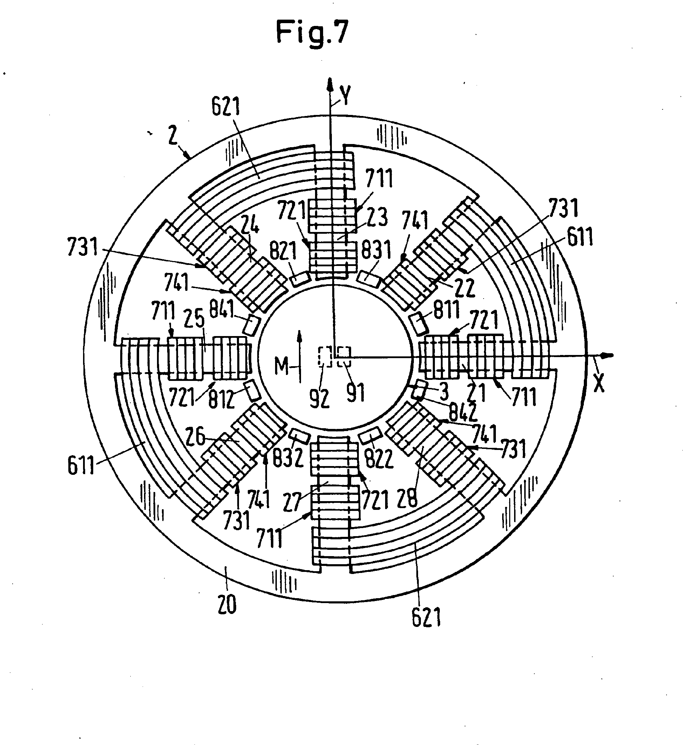

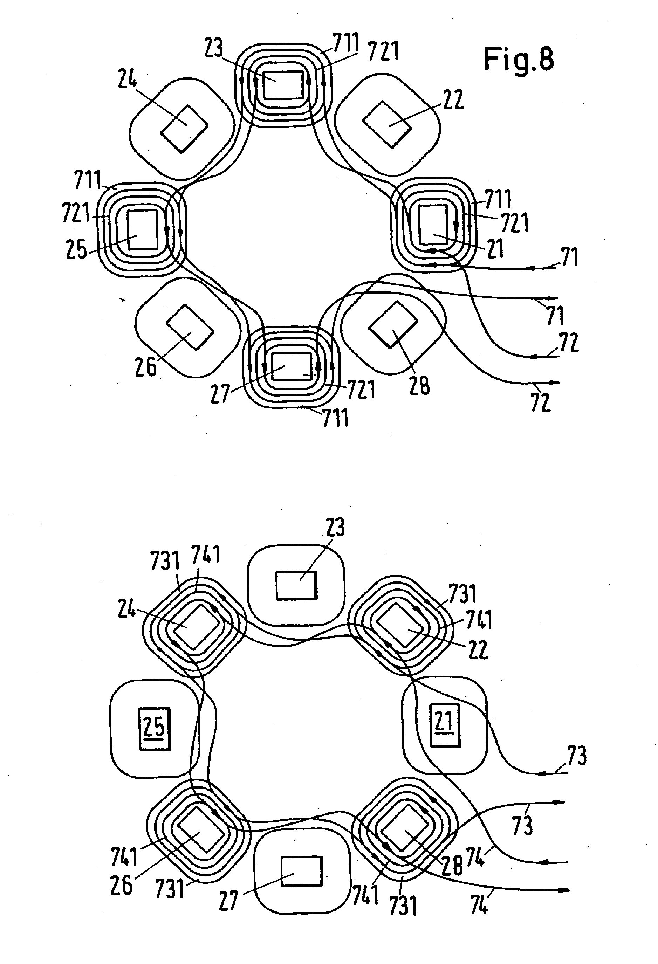

Fig. 7 zeigt in einer Aufsicht den Stator 2 einer Variante des ersten Ausführungsbeispiels. Im Folgenden wird nur auf die Unterschiede näher eingegangen. Bei dieser Variante mit ebenfalls vier Steuerphasen und zwei Antriebsphasen umfasst die Steuerwicklung insgesamt sechzehn konzentrierte Steuerspulen 711,721,731,741, wobei auf jedem Statorzahn 21-28 jeweils zwei Steuerspulen vorgesehen sind. Jeweils vier Steuerspulen, die um vier verschiedene Statorzähne gewickelt sind, sind zu einem Strang 71-74 der Steuerwicklung zusammengeschaltet und gehören somit zur gleichen Steuerphase (diese Steuerspulen haben wiederum jeweils identische Bezugszeichen). Die Verschaltung der einzelnen Steuerspulen ist der schematischen Darstellung in Fig. 8 zu entnehmen. Der erste Strang 71 (siehe Fig. 8, oben) der Steuerwicklung wird durch Zusammenschalten jeweils einer der beiden Steuerspulen realisiert, die auf dem ersten, dritten, fünften und siebten Statorzahn 21,23,25,27 angeordnet sind. Diese Steuerspulen sind mit dem Bezugszeichen 711 bezeichnet. Der zweite Strang 72 der Steuerwicklung wird durch Zusammenschalten der jeweils anderen Steuerspulen auf dem ersten, dritten, fünften und siebten Statorzahn 21,23,25,27, die also nicht zum ersten Strang 71 gehören, realisiert. Diese Steuerspulen sind mit dem Bezugszeichen 721 bezeichnet. Die Steuerspulen 721 des zweiten Strangs 72 befinden sich also auf denselben Statorzähnen wie die Steuerspulen 711 des ersten Strangs 71. Der erste und der zweite Strang sind somit im wesentlichen durch identische Anordnung und Zusammenschaltung ihrer jeweiligen Steuerspulen realisiert.

Analoges gilt für die Steuerspulen des dritten und vierten Strangs 73 und 74, die mit den Bezugszeichen 731 bzw. 741 bezeichnet sind. (Fig. 8, unten). Diese sind jeweils auf dem zweiten, vierten, sechsten und achten Statorzahn 22,24,26,28 angeordnet.

Die Steuerspulen sind also derart zu den einzelnen Strängen der Steuerwicklung zusammengeschaltet, dass in Umfangsrichtung des Stators 2 gesehen jeweils die um unmittelbar benachbarte Statorzähne gewickelten Steuerspulen zu verschiedenen Strängen der Steuerwicklung gehören.

Die jeweils beiden Steuerspulen, die auf demselben Statorzahn angeordnet sind, können nebeneinander angeordnet sein, so wie dies in Fig. 7 dargestellt ist. Vorzugsweise, insbesondere bei der weiter hinten beschriebenen Bauform des Tempelmotors, sind jeweils die beiden Steuerspulen, die um denselben Statorzahn gewickelt sind, aufeinander gewickelt, sodass die äussere Steuerspule die innere Steuerspule umgibt. Diese Anordnung ist in Fig. 8 schematisch gezeigt. Die vier Steuerspulen, die zum gleichen Strang der Steuerwicklung zusammengeschaltet sind, sind immer nur die äusseren oder nur die inneren Steuerspulen. So sind z. B. die Steuerspulen 711 des ersten Strangs 71 nur äussere Spulen, während die Steuerspulen 721 des zweiten Strangs nur innere Spulen sind. Die einzelnen Steuerspulen sind bevorzugt so ausgebildet, dass der ohmsche Widerstand des Strangs, der von äusseren Steuerspulen gebildet wird, z. B. Strang 71, entweder gleich gross (symmetrische Ausgestaltung) oder grösser (asymmetrische Ausgestaltung) ist, als der ohmsche Widerstand des Strangs der von den inneren Steuerspulen gebildet wird, z. B. Strang 72.

Die zweiphasige Antriebswicklung ist in gleicher Weise ausgestaltet wie dies in Fig. 5 dargestellt ist und im Zusammenhang mit dem ersten Ausführungsbeispiel bereits erläutert wurde.

Die in Fig. 7 und Fig. 8 gezeigte Ausgestaltung der Steuerwicklung hat den folgenden Vorteil: Im Normalbetrieb wird die Position des Rotors mit allen vier Steuerphasen, also mit allen sechszehn Steuerspulen, geregelt. Da auf jedem Statorzahn zwei Steuerspulen angeordnet sind, braucht durch diese jeweils nur der halbe Strom zu fliessen, der nötig wäre, um das gleiche Feld mit nur einer Steuerspule auf diesem Statorzahn zu erzeugen. Zweimal der halbe Strom ist jedoch von den Verlusten her günstiger als einmal der ganze Strom. Fällt nun eine Steuerphase aus, beispielsweise der erste Strang 71, so kann dies in einfacher Weise dadurch kompensiert werden, dass der Phasenstrom im Strang 72 der zweiten Steuerphase verdoppelt wird. Dies erfolgt automatisch durch die Regelung. Durch diese Ausgestaltung mit zwei Steuerspulen pro Statorzahn, die zu unterschiedlichen Steuerphasen gehören und die im Normalbetrieb beide aktiv sind, wird eine heisse Redundanz realisiert. Das "Notsystem" ist bereits im Normalbetrieb aktiv, und der Fehler muss nicht erst erkannt werden, bevor er kompensiert werden kann.

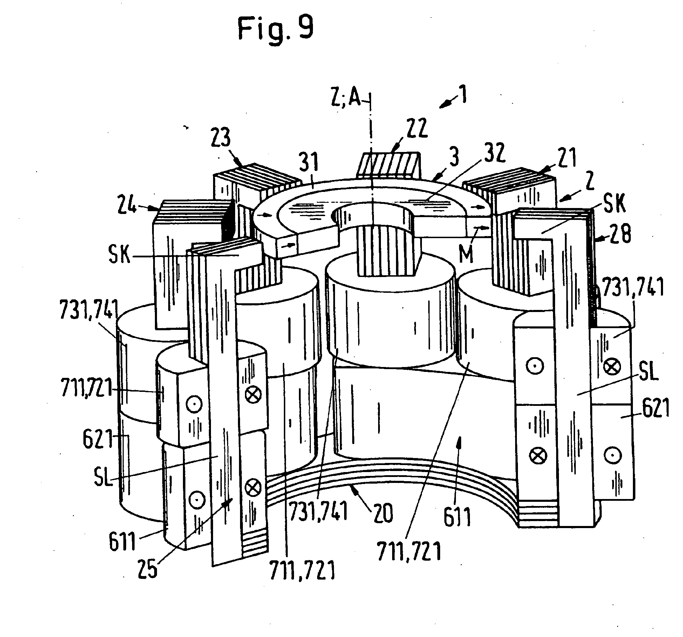

Fig. 9 zeigt in einer perspektivischen Darstellung und teilweise im Schnitt ein zweites Ausführungsbeispiel des erfindungsgemässen Drehantriebs 1, das als sogenannter Tempelmotor ausgestaltet ist. Ein Tempelmotor wird beispielsweise in der bereits zitierten WO-A-98/11650 offenbart (siehe dort Fig. 8k und zugehörige Textpassagen). Der Stator 2 des Tempelmotors weist mehrere, hier acht, durch einen Rückschluss 20 verbundene Statorzähne 21-28 auf (In Fig. 9 sind nur sechs Statorzähne 21-25,28 zu erkennen). Der Rückschluss 20, der die Statorzähne 21-28 magnetisch miteinander koppelt, ist aus einem ferromagnetischen Marterial, vorzugsweise Eisen, hergestellt. Jeder Statorzahn 21-28 ist L-förmig ausgebildet mit einem längeren Schenkel SL und einem kürzeren Schenkel SK. Der längere Schenkel SL erstreckt sich jeweils in der axialen Richtung und der kürzere Schenkel SK verläuft radial nach innen, also auf den Rotor zu. Die Antriebs- und Steuerspulen 611,621,711,721,731,741 sind um die längeren Schenkel SL der Statorzähne 21-28 gewickelt. Die Ausführungsformen des Stators gemäss Fig. 2 und Fig. 7 ("planare" Ausgestaltung) einerseits und Fig. 8 (Tempelmotor) andererseits sind auf identische Weise ansteuerbar, funktionell gleichwertig, insbesondere von ihrer elektrischen Wirkungsweise, und haben insbesondere dieselbe Wirkung auf den permanentmagnetischen Rotor 3. Die L-förmigen Statorzähne 21-28 sind vom Funktionsprinzip äquivalent zu den "geraden" Statorzähnen 21-28 wie sie in Fig. 2 und Fig. 7 gezeigt sind.

Die Wicklung, Anordnung und Verschaltung der Antriebsspulen 611 und 621 ist die gleiche wie sie in Fig. 5 gezeigt ist und bereits erläutert wurde. Die Stränge 71-74 der Steuerphasen können entweder mit acht diskreten Steuerspulen 711,721,731,741, also einer pro Statorzahn, ausgestaltet sein, die wie in Fig. 4 gezeigt angeordnet und verschaltet sind, oder mit sechzehn diskreten Steuerspulen 711,721,731,741, also zwei pro Statorzahn, die gemäss Fig. 8 angeordnet und verschaltet sind. Bei der Variante mit zwei Steuerspulen pro Statorzahn können diese beiden Steuerspulen bezüglich der axialen Richtung benachbart, oder, was bevorzugt ist, aufeinander gewickelt sein, so wie dies weiter vorne bereits erläutert wurde. Der Tempelmotor gemäss Fig. 9 mit vier Steuerphasen und zwei Antriebsphasen ist funktionell äquivalent zum ersten Ausführungsbeispiel bzw. seiner Variante. Die diesbezüglichen Erläuterungen gelten in sinngemäss gleicher Weise auch für die Ausgestaltung als Tempelmotor gemäss Fig. 9.

Natürlich kann der Tempelmotor auch mit einer anderen Anzahl von Statorzähnen und/oder mit anders ausgestalteter Antriebswicklung und/oder Steuerwicklung ausgestaltet sein. Die Positionssensoren und die Feldsensoren sind in Fig. 9 nicht dargestellt. Falls der erfindungsgemässe Drehantrieb Teil einer Blutpumpe ist, so ist er vorzugsweise als Tempelmotor ausgestaltet, wie weiter hinten noch erläutert wird.

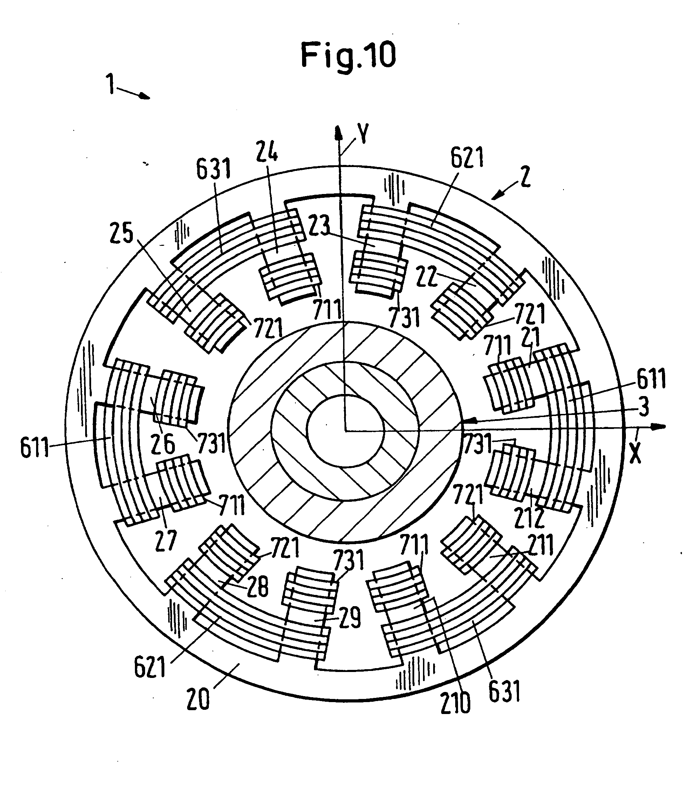

Fig. 10 veranschaulicht in einer Fig. 2 entsprechenden Darstellung ein drittes Ausführungsbeispiel der Erfindung, bei welchem der Drehantrieb 1 genau drei Antriebsphasen und genau drei Steuerphasen aufweist. Im Folgenden wird nur auf die Unterschiede zu den ersten beiden Ausführungsbeispiele näher eingegangen. Ansonsten gelten die voranstehenden Erläuterungen in sinngemäss gleicher Weise auch für das dritte Ausführungsbeispiel. Insbesondere sind identische oder von der Funktion her gleichwertige Teile mit den gleichen Bezugszeichen bezeichnet.

Bei dem dritten Ausführungsbeispiel hat der Stator 2 zwölf Statorzähne 21-29,210,211,212, zwischen denen der Rotor 3 magnetisch lagerbar ist. Entsprechend den drei Antriebsphasen weist die Antriebswicklung drei Stränge auf, von denen jeder durch die Verschaltung von zwei diskreten Antriebsspulen 611,621,631 realisiert ist. Insgesamt sind also sechs Antriebsspulen 611,621,631 vorgesehen, die jeweils als konzentrierte Spulen ausgebildet sind. Jede Antriebsspule 611,621,631 ist in sinngemäss gleicher Weise wie im Zusammenhang mit Fig. 2 erläutert um jeweils zwei unmittelbar benachbarte Statorzähne gewickelt. Jeweils die beiden sich diametral gegenüberliegenden Antriebsspulen 611 bzw. 621 bzw. 631 sind zu einem Strang der Antriebswicklung verschaltet, das heisst die beiden mit dem Bezugszeichen 611 versehenen Antriebsspulen bilden den ersten Strang der Antriebswicklung, die beiden mit dem Bezugszeichen 621 bezeichneten Antriebsspulen bilden den zweiten Strang der Antriebswicklung und die beiden mit dem Bezugszeichen 631 bezeichneten Antriebsspulen bilden den dritten Strang der Antriebswicklung.

Die dreiphasige Steuerwicklung umfasst zwölf diskrete Steuerspulen 711,721,731, die als konzentrierte Spulen ausgebildet sind, und von denen jeweils vier Steuerspulen einen Strang der Steuerwicklung bilden, also zu derselben Steuerphase gehören. Auf jedem Statorzahn 21-212 ist jeweils eine Steuerspule vorgesehen. Die vier mit dem Bezugszeichen 711 bezeichneten Steuerspulen, die um den ersten, vierten, siebten bzw. zehnten Statorzahn 21,24,27,210 gewickelt sind, sind zum Strang der ersten Steuerphase verschaltet, die vier mit 721 bezeichneten Steuerspulen auf dem zweiten, fünften, achten bzw. elften Statorzahn 22,25,28,211 bilden den Strang der zweiten Steuerphase, und die vier mit 731 bezeichneten Steuerspulen auf dem dritten,sechsten, neunten bzw. zwölften Statorzahn 23,26,29,212 bilden den Strang der dritten Steuerphase.

Die Polpaarzahl der Antriebswicklung ist p=1 und die Polpaarzahl der Steuerwicklung ist gleich zwei, also gleich p+1.

Die Positionssensoren (in Fig. 10 nicht dargestellt) sind bevorzugt jeweils zwischen zwei benachbarten Statorzähnen angeordnet, in gleicher Weise wie es bereits vorne erläutert wurde. Die Feldsensoren (in Fig. 10 nicht dargestellt) sind vorzugsweise analog wie in Fig. 2 und Fig. 3 gezeigt, axial versetzt zur X-Y-Ebenen unterhalb oder oberhalb des Rotors 3 angeordnet.

Es versteht sich, dass auch bei dem dritten Ausführungsbeispiel die Stelleinrichtung 4 (Fig. 1) für die Versorgung der einzelnen Stränge der Antriebs- und der Steuerwicklung so ausgestaltet ist, dass jede Antriebsphase und jede Steuerphase vollkommen unabhängig von den anderen Phasen angesteuert bzw. betrieben werden kann. Vorzugsweise ist auch hier für jeden der drei Stränge der Antriebswicklung und für jeden der drei Stränge der Steuerwicklung jeweils ein separater bipolarer Leistungsverstärker 41 (Fig. 1, Fig. 6) vorgesehen, sodass der Phasenstrom (oder die Phasenspannung) für jeden Strang der Antriebswicklung und für jeden Strang der Steuerwicklung unabhängig von den Phasenströmen (oder den Phasenspannungen) für die anderen Stränge regelbar ist. Bezüglich weiterer Details der fehlertoleranten Ausgestaltung mit drei Antriebsphasen bzw. mit drei Steuerphasen sei auf die beiden bereits zitierten europäischen Patentanmeldungen Nr. 99810553.0 und Nr. 99810760.1 verwiesen.

Es versteht sich, dass auch das dritte Ausführungsbeispiel als Tempelmotor (siehe Fig. 9) ausgestaltet sein kann.

Abweichend von den hier beschriebenen Ausführungsbeispielen ist es auch möglich, die Steuerspulen und/oder die Antriebsspulen in Form von verteilten Wicklungen auszugestalten.

Als Kontrollvorrichtung 5 (Fig. 1) eignet sich prinzipiell jede Kontrollvorrichtung, mit der ein lagerloser Motor beteibbar ist, vorausgesetzt die Kontrollvorrichtung 5 wird so modifiziert, dass sie ein vollkommen unabhängiges Ansteuern und bipolares Betreiben jeder einzelnen Antriebsphase und jeder einzelnen Steuerphase ermöglicht.

Im Folgenden wird jedoch noch ein besonders bevorzugtes Ausführungsbeispiel für die Kontrollvorrichtung 5 beschrieben, das sich durch seine zusätzliche hohe Fehlertoleranz auszeichnet. Dabei wird auf den Fall Bezug genommen, dass die Antriebswicklung 6 genau zweiphasig und die Steuerwicklung 7 genau vierphasig ausgestaltet ist. Dies ist jedoch keine zwingende Voraussetzung. Diese Kontrollvorrichtung 5 kann in sinngemäss gleicher Weise auch für eine andere Anzahlen von Antriebsphasen und/oder Steuerphasen ausgelegt werden.

Fig. 11 zeigt in einer Blockdarstellung den prinzipiellen Aufbau des Ausführungsbeispiels der Kontrollvorrichtung 5, wobei Kommunikationsverbindungen oder Verbindungen zum Signal- und Datentransfer sowie Versorgungsverbindungen jeweils durch Pfeile angedeutet sind. Die Kontrollvorrichtung umfasst zwei fast identische Kontrollsysteme 5a,5b, von denen jedes alleine und unabhängig von dem anderen Kontrollsystem den lagerlosen Motor 1 (hier symbolisch als Blutpumpe 10 dargestellt) betreiben kann. Im normalen fehlerfreien Betriebszustand sind beide Kontrollsysteme 5a,5b aktiv und regeln gemeinsam den Betrieb des lagerlosen Motors 1. Im Falle eines Fehlers kann jedoch jedes der beiden Kontrollsysteme unmittelbar alleine den Betrieb des lagerlosen Motors 1 kontrollieren und regeln, das heisst der ordnungsgemässe Betrieb des Motors 1 ist sowohl bezüglich des Antriebs als auch bezüglich der Lagerung mit nur einem der beiden Kontrollsysteme 5a,5b möglich. Diese heisse Redundanz bedeutet eine erhebliche Steigerung der Betriebssicherheit, was insbesondere für Blutpumpen ein grosser Vorteil ist.

Je nach auftretendem Fehler ist es aber auch möglich, nur einen Teil eines Kontollsystems 5a,5b zu deaktivieren, was weiter hinten noch erläutert wird.

Kurzfristig, das heisst für eine Zeitspanne von mindestens drei Millisekunden, kann der lagerlose Motor sogar beim Ausfall beider Kontrollsysteme 5a,5b weiterarbeiten, ohne dass es zu grösseren Störungen kommt, beispielsweise zu einem körperlichen Kontakt zwischen Rotor und Stator. Dies hat den Vorteil, dass bei einem Ausfall beider Kontrollsysteme 5a,5b genügend Zeit verbleibt, um durch einen Reset wenigstens eines der beiden Kontrollsysteme 5a, 5b neu zu starten; denn für einen solchen Reset wird typischerweise nur eine Zeit von ungefähr einer Millisekunde benötigt. Ist der Reset für mindestens ein Kontrollsystem erfolgreich, so kann der ordnungsgemässe Betrieb fortgesetzt werden.

Von den beiden Kontrollsystemen 5a,5b wird eines, hier das Kontrollsystem 5a, als Master betrieben und das andere Kontrollsystem 5b als Slave. Jedes Kontrollsystem 5a,5b umfasst zumindest die folgenden Komponenten: mindestens zwei Positionssensoren 81,82 bzw. 83,84, die jeweils ein Zwei-Achsen-Messsystem 80a,80b zur Bestimmung der radialen Position des Rotors 3 bilden; Mittel zur Bestimmung des Rotorwinkels, die hier mindestens einen Feldsensor 91,92 pro Kontrollsystem umfassen; mindestens drei separate, in einem Leistungsteil 4a,4b vorgesehene Leistungsverstärker 41 zur Versorgung der einzelnen Stränge der Antriebs- und der Steuerwicklung; sowie eine Signalverarbeitungs- und Regeleinrichtung 50a,50b, welche die Sensorsignale verarbeitet, die Regelung des Antriebs und der Position des Rotors durchführt, und das Leistungsteil 4a,4b mit den Leistungsverstärkern 41 ansteuert. Die Leistungsverstärker 41 jedes Kontrollsystem 5a,5b steuern mindestens eine Antriebsphase und mindestens zwei Steuerphasen an, sodass jedes Kontrollsystem alleine den lagerlosen Motor 1 betreiben kann. Das Leistungsteil 4a versorgt beispielsweise den Strang 61 (siehe Fig. 5) der Antriebswicklung 6 sowie die beiden Stränge 71,73 (siehe Fig. 4 und Fig. 8) der Steuerwicklung 7, während das Leistungsteil 4b den Strang 62 der Antriebswicklung 6 sowie die beiden Stränge 72,74 der Steuerwicklung 7 speist. Jedes Kontrollsystem kontrolliert also jeweils einen der Stränge 71,72 der Steuerwicklung, die im oberen Bild der Fig. 4 bzw. der Fig. 8 dargestellt sind, und einen der Stränge 73,74 der Steuerwicklung, die im unteren Bild der Fig. 4 bzw. der Fig. 8 dargestellt sind. Jedes Kontrollsystem 5a,5b wird mit zwei Spannungen, hier einer geregelten Gleichspannung von 3V (3V_A bzw. 3V_B) und einer Gleichspannung von 12V (12V_A bzw, 12V_B) versorgt.

Da die Signalverarbeitungs- und Regeleinrichtung 50a,50b üblicherweise auf einem Chip bzw. einem Elektronikprint realisiert ist, wird sie im Folgenden kurz als DSP-Board bezeichnet (DSP: Digital Signal Processor). Auch die übrigen Komponenten der Kontrollsysteme 5a,5b sind vorzugsweise in Form von Elektronikprints bzw. Chips realisiert, wie dies die Darstellungen insbesondere in den Fig. 11, 15-18 andeuten.

In beiden Kontrollsystemen 5a,5b können einzelne Komponenten abgeschaltet werden, wodurch auf eine Vielzahl von Fehlern reagiert werden kann, und zwar unter Erhaltung der optimalen bei dem jeweiligen Fehler noch möglichen Betriebsbedingungen. Damit die Kontrollvorrichtung 5 auf verschiedene Fehlerzustände jeweils optimal reagieren kann, verfügt jedes Kontrollsystem 5a,5b über die Prozess- und Systemdaten, die zum Betrieb notwendig sind (Software usw.). Zusätzlich können die beiden Kontrollsysteme 5a,5b Signale miteinander austauschen und können sich gegenseitig über eine parallele oder serielle Datenverbindung (paralleler oder serieller Datenbus) überwachen sowie überprüfen.

Während des fehlerfreien Normalbetriebs können die beiden Kontrollsysteme 5a,5b Synchronisationssignale miteinander austauschen, um die Schaltzyklen der Leistungsverstärker 41 (H-Brücken) und die Anregungsfrequenzen der Positionssensoren miteinander zu synchronisieren, damit hier keine Störungen auftreten. Die Verbindungen für den Austausch der Synchronisationssignale können im Fehlerfall sofort geöffnet werden.

Im Folgenden werden die wesentlichen Komponenten der Kontrollsysteme 5a,5b im Einzelnen beschrieben.

Fig. 12 zeigt ein Blockschaltbild der Signalverarbeitungs- und Regeleinrichtung 50a (DSP-Board) des Kontrollsystems 5a (Master), die im wesentlichen identisch ist mit derjenigen des Kontrollsystems 5b (Slave). Daher gelten die Erläuterungen auch für das Kontrollsystem 5b. Der Buchstabe "a" bzw. "b" am Ende eines Bezugszeichens zeigt an, ob das bezeichnete Element zum Master 5a bzw. zum Slave 5b gehört.

Das DSP-Board 50a ist verantwortlich für die Regelung der Position des Rotors 3 und der Phasenströme für die Antriebs- und Steuerphasen, das Lesen und Interpretieren sämtlicher Informationen und das Aussenden von Kontrollsignalen an die verschiedenen Komponenten. Das DSP-Board 50a umfasst als "Gehirn" eine Prozessoreinheit, nämlich den digitalen Signalprozessor (DSP) 51a. Ferner sind auf dem DSP-Board folgende Komponenten vorgesehen:
eine mit dem Bezugszeichen 52a versehene Logik, die als FPGA (Field Programmable Gate Array) bezeichnet wird, einen Flash-Speicher 53a, zwei Analog-Digital-Wandler 54a (ADC), ein Taktgeber 55a, ein Linearregler (linear regulator) 56a, sowie einen Watchdog 57a. Zudem sind zwei Interprozessorverbindungen 58, 59 als Kommunikationsmittel vorgesehen, die jeweils einen Treiber oder Transceiver 581,591a umfassen. Die Versorgungsspannungen sind durch die kleinen Pfeile mit der Beschriftung 3V bzw. 1.8V/3V symbolisch dargestellt.

Das gesamte System wird in an sich bekannter Weise über ein sogenanntes transkutanes Energie-Transfersystem (Transcutaneous Energy Transfer System, TETS) mit Energie versorgt. Über das TETS können auch Daten übermittelt werden wie z. B. Downloads oder Programmänderungen. Das TETS ist über eine Kommunikationsschnittstelle KS mit dem DSP verbunden.

Der DSP 51a,51b ist beispielsweise ein Prozessor vom Typ TMS320VC5402 der Firma Texas Instruments, der z. B. mit 100 MHz oder mit 50 MHz getaktet wird. Der DSP benötigt zwei Versorgungsspannungen, nämlich 3 V und 1.8 V, auf deren Bereitstellung noch eingegangen wird. Die 3V-Spannung wird für die Ausgangs-Pins benötigt, während die 1.8 V zur Speisung der Kemeinheit dienen. Der obengenannte DSP 51a,51b hat einen 16K×16 Bit RAM-Speicher mit Zweifach-Zugriff, der es erlaubt, das gesamte Regelprogramm für den lagerlosen Motor im DSP 51a,51b selbst durchzuführen, ohne auf den externen Flash-Speicher 53a,53b zuzugreifen.

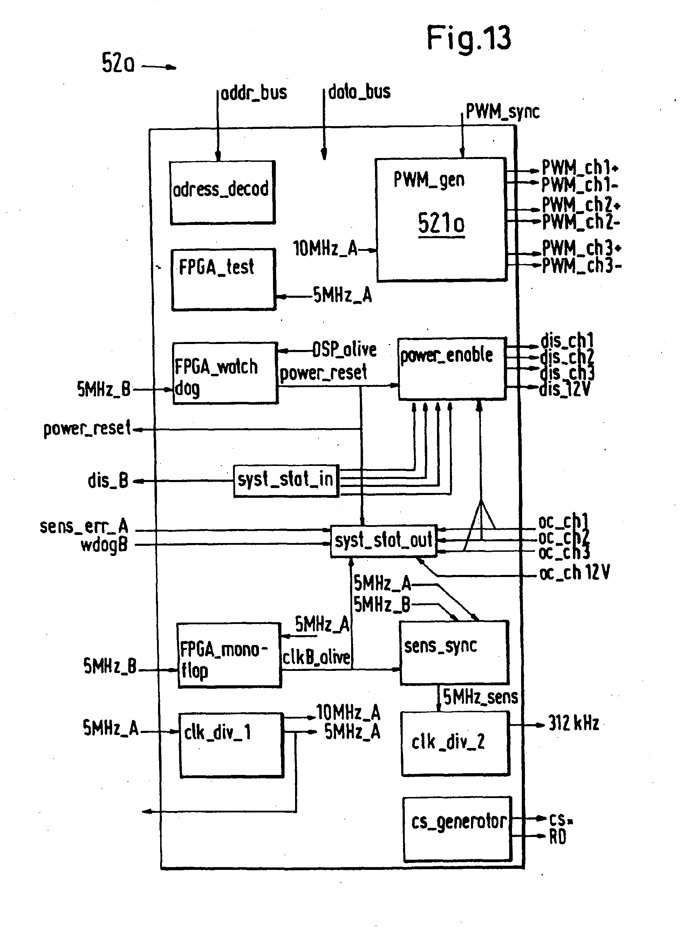

Das FPGA 52a,52b beinhaltet die Verbindungslogik (Glue Logic) und dient zum Generieren der Pulsweitenmodulation (PWM), mit welcher die Leistungsverstärker 41 angesteuert werden. Das FPGA 52a,52b ist beispielsweise aus der 42MX Familie der Firma Actel, weil diese sehr klein (geringer Platzbedarf) sind und zudem wenig Energie verbrauchen. Das FPGA 52a,52b umfasst zum einen die Verbindungslogik, welche der DSP 51a,51b zur Dekodierung von Adressen und zum Überwachen braucht, und zum anderen einen PWM-Generator 521a (siehe Fig. 13), welcher die PWM-Signale zum Ansteuern der Schalttransistoren T (siehe Fig. 6) der Leistungsverstärker 41 generiert.

In Fig. 13 ist ein Blockdiagramm des FPGA 52a dargestellt, in welchem die einzelnen Module des FPGA 52a des Masters 5a gezeigt sind. Das Modul adress_decod kommuniziert mit dem Adressbus des DSP 51a (Pfeil "addr_bus") und decodiert die Adressen, die von dem DSP 51a übermittelt werden. Das Modul FPGA_test ist ein Zähler, der von dem Taktgeber 55a des gleichen Kontrollsystems 5a getaktet wird (Signal 5MHz_A). Der DSP 51a kann diesen Zähler auslesen. Wenn sich sein Wert nicht mehr ändert, entscheidet der DSP 51a, dass das FPGA 52a ausgefallen ist und schaltet in den entsprechenden Fehlermodus (siehe weiter hinten). Das Modul FPGA_watchdog ist eine Überwachungseinheit (interner Watchdog) für das FPGA 52a. Dieses Modul ist ein rücksetzbarer Zähler, der von dem Taktgeber 55b des anderen Kontrollsystems 5b getaktet wird (Signal 5MHz_B) und während jedem Software-Zyklus durch ein Signal DSP_alive zurückgesetzt wird. Das Signal DSP_alive wird von dem DSP 51a des gleichen Kontrollsystems 5a generiert. Falls dieses Signal ausbleibt, weil beispielsweise der DSP 51a ausgefallen ist oder der Software-Zyklus nicht korrekt durchlaufen wird, so generiert das Modul FPGA_watchdog ein Signal power_reset, welches einerseits über ein Modul power_enable (siehe unten) die 12V-Versorgung der Leistungsverstärker 41 vorübergehend abschaltet (dis_12V) und andererseits einen Reset an den DSP 51a gibt. Nach erfolgreichem Reset wird die 12V-Versorgung wieder eingeschaltet. Der gesamte Reset-Vorgang kann in weniger als einer Millisekunde durchgeführt werden, sodass im Falle eines erfolgreichen Resets keine Beeinflussung der ordnungsgemässen Lager- und Antriebsfunktion auftritt.

Das Modul syst_stat_in ist ein Kontrollwort, das nur von dem DSP 51a geändert werden kann. Durch Änderung spezifischer Bits dieses Worts kann der DSP 51a das Abschalten der 3 V-Versorgungsspannung des Kontrollsystems 5b bewirken (dis_B). Ferner kann das Modul syst_stat_in die eigenen Phasen, das heisst die einzelnen Leistungsverstärker 41 derjenigen Antriebs- und Steuerphasen, die vom Kontrollsystem 5a angesteuert werden, über das Modul power_enable, welches mit dem Modul syst_stat_in verbunden ist, jeweils einzeln deaktivieren (dis_ch1,2,3) oder aktivieren. Das power_enable Modul kann zusätzlich die gesamte 12V-Versorgungsspannung der Leistungsverstärker 41, die vom Kontrollsystem 5a angesteuert werden, deaktivieren (dis_12V). Das Modul syst_stat_out enthält verschiedene Informationen, z. B. ob und falls ja, in welchem Leistungsverstärker 41 (H-Brücke) ein Überstrom fliesst (oc_ch1,2,3), ob in der 12V-Versorgungsspannung der Leistungsverstärker 41 ein Überstrom, z. B. aufgrund eines Kurzschlusses, fliesst (oc_ch12V), ob eine Fehlfunktion in der Anregung der Sensoren des Kontrollsystems 5a vorliegt (sens_err_A) oder ob der Watchdog 57b des anderen Kontrollsystems 5b einen Fehler meldet (wdog_B), der anzeigt, das der DSP 51b des anderen Kontrollsystems 51b nicht mehr oder nicht korrekt arbeitet. Das Modul FPGA_monoflop ist ein Schaltkreis, der den Taktgeber 55b des anderen Kontrollsystems 5b überwacht (5MHz_B) und ein Signal erzeugt (clkB_alive), das anzeigt, ob dieser Taktgeber 550. noch arbeitet. Das Modul sens_sync synchronisiert die Anregungsfrequenzen (5MHz_A, 5MHz_B) für die Positionssensoren der beiden Kontrollsysteme 5a, 5b und hat als Ausgangssignal die synchronisierte Anregungsfrequenz (5MHz_sens). Diese wird im Modul clk_div_2 durch sechszehn dividiert und als Anregungsfrequenz (312 kHz) für die Positionssensoren bereitgestellt. Im normalen Betriebszustand gibt der Master 5a den Takt für die Messsysteme (Positions- und Feldsensoren) beider Kontrollsysteme 5a,5b vor. Im Fehlerfall, wenn z. B. dieses Taktsignal des Masters 5a ausfällt, muss der Slave 5b sofort auf seinen eigenen Taktgeber zurückgreifen können. Diese Funktion wird von dem Modul sens_sync übernommen, das von dem dkB_alive Signal des FPGA_monoflop Moduls angesteuert wird. Das Modul clk_div_1 dividiert das Taktsignal an seinem Eingang (50MHz_A), das identisch mit dem Taktsignal für den DSP 51a ist (50 MHz oder 100 MHz Taktfrequenz), durch zehn und durch fünf und stellt so an seinem Ausgang das 5 MHz- und ein 10 MHz-Taktsignal für das Kontrollsystem 5a bereit (10MHz_A, 5MHz_A). Das Modul cs_generator ist ein Chip-Select Generator, der die Schaltkreise jedes Kontrollsystems addressiert, sodass der DSP 51a über einen Bus mit diesen kommunizieren kann.

Der PWM-Generator 521a (Modul PWM_gen) wird mit der 10 MHz-Taktfrequenz getaktet (10MHz_A) und steuert die einzelnen Schalttransistoren T (siehe Fig. 6) der H-Brücken der Leistungsverstärker 41 an, um so die jeweils korrekte Phasenspannung an den zugehörigen Strang der Antriebs- oder der Steuerwicklung anzulegen. Für jeden separaten Leistungsverstärker 41 sind zwei Ausgänge am PWM-Generator 521a vorgesehen (PWM_chx+ und PWM_chx-, mit x=1 oder 2 oder 3), wobei der Plus-Ausgang die darstellungsgemäss (Fig. 6) oberen Schalttransistoren T ansteuert und der Minus-Ausgang die unteren Schalttransistoren T. Der PWM-Generator 521a erhält ferner ein Synchronisationssignal (PWM_sync) das gewährleistet, dass die beiden PWM-Generatoren 521a,521b von Master 5a und Slave 5b synchron und nicht gegeneinander arbeiten. Der DSP 51a, welcher die regelungstechnischen Sollwerte für die Phasenspannungen der beiden Steuerphasen und der Antriebsphase berechnet, übermittelt diese Daten über einen Datenbus (data_bus) an den PWM-Generator 521a. Über diesen Datenbus erhält das FPGA 52a auch weitere Daten, die es benötigt.

Der Flash-Speicher 53a,53b ist ein CMOS-Permanentspeicher (non volatile memory), der mit der Versorgungsspannung von 3V arbeitet. In ihm ist das gesamte Programm für den Betrieb des lagerlosen Motors gespeichert. Die Kapazität dieses Flash-Speichers 53a,53b beträgt z. B. 256Kx16 Bit. Der Flash-Speicher 53a,53b wird nur benötigt, wenn das gesamte System (Drehantrieb 1 inklusive Kontrollvorrichtung 5) neu gestartet wird, wenn der DSP 51a,51b komplett zurückgesetzt wird (Reset), oder wenn während des Betriebs Updates des Programms gespeichert werden sollen. Im Falle des Neustarts oder eines Resets des DSP 51a,51b wird das gesamte Programm für den Betrieb des lagerlosen Motors aus dem Flash-Speicher 53a über einen Bus in den internen RAM-Speicher des DSP 51a,51b geladen. Soll während des Betriebs ein Update des Programms erfolgen, so wird dieses in den Flash-Speicher 53a,53b geschrieben, während der DSP 51a,51b noch mit der älteren, in seinem internen RAM gespeicherten Programmversion den Drehantrieb 1 betreibt. Nachdem das neue Programm (Update) komplett in den Flash-Speicher 53a,53b geladen, überprüft und validiert wurde, erhält der DSP 51a,51b einen Reset-Befehl und übernimmt dann das neue Programm aus dem Flash-Speicher 53a,53b in seinen internen RAM-Speicher, um es für den weiteren Betrieb zu verwenden. Während des normalen Betriebs befindet sich der Flash-Speicher 53a,53b in einem Schlaf-Modus, in welchem er fast keine Energie verbraucht.

Die beiden ADCs 54a,54b, die jedes der beiden Kontrollsystem 5a,5b hat, sind beispielsweise jeweils Vier-Kanal Analog-Digital-Wandler. Somit kann jedes Kontrollsystem 5a,5b acht analoge Signale lesen, wobei jeweils zwei simultan gelesen werden können. Von diesen acht analogen Eingängen werden drei für die drei Phasenströme von den drei Strommessgeräten 411 in den H-Brücken der drei Leistungsverstärker 41 (siehe Fig. 6) genutzt, zwei für die beiden Positionssignale von den beiden Positionssensoren 81,82 bzw. 83,84 sowie zwei für die beiden Signale (Sinus und Cosinus) des Feldsensors 91 bzw. 92. Der noch verbleibende Eingang kann für andere Zwecke genutzt werden. Die ADCs 54a,54b kommunizieren mit dem DSP 51a,51b über zwei serielle Verbindungen, die unabhängig von dem parallelen Datenbus sind, über den die beiden DSPs 51a,51b miteinander und mit dem Flash-Speicher 53a,53b kommunizieren, und damit auch unabhängig von dem FPGA 52a,52b.

Der Taktgeber 55a,55b generiert mittels eines Kristalls eine Taktfrequenz für das Kontrollsystem 5a,5b. Der Taktgeber 55a,55b ist beispielsweise ein Parallel-Resonanz-Kristall.

Der Linearregler 56a erzeugt aus der Versorgungsspannung von 3V die Gleichspannung von 1.8 V für den DSP 51a,51b. Prinzipiell sind hierfür auch Gleichstromkonverter (DC-DC converter) geeignet. Diese verbrauchen zwar etwas weniger Energie, benötigen aber viel mehr Platz auf dem Elektronikprint als der Linearregler 56a. Letzterer ist daher bevorzugt.

Mit dem Watchdog 57a,57b, beispielsweise vom Typ TPS3305-18 der Firma Texas Instruments, wird der DSP 51a,51b überwacht und speziell das Vorhandensein der beiden Spannungen von 3V und 1.8 V. Der Watchdog 57a,57b muss regelmässig von dem DSP 51a,51b angesteuert (getriggert) werden. Bleibt dieses Signal aus, so wird nach einer vorgebbaren Zeit von beispielsweise 1.6 Sekunden ein Reset-Impuls generiert. Mittels der Watchdogs 57a,57b können sich die beiden DSPs 51a,51b im normalen Betriebszustand gegenseitig überwachen. Falls ein DSP, beispielsweise der DSP 51a während einer vorgebbaren Zeit nicht auf den Watchdog zugreift und einen Reset auslöst, kann der andere DSP 51b diesen Zustand erfassen und darauf reagieren. Falls der Fehler nach dem Reset nicht verschwunden ist und wiederum einen Reset zur Folge hat, wird die Versorgungsspannung des DSP 51a durch den DSP 51b abgeschaltet, wodurch dieser deaktiviert wird.

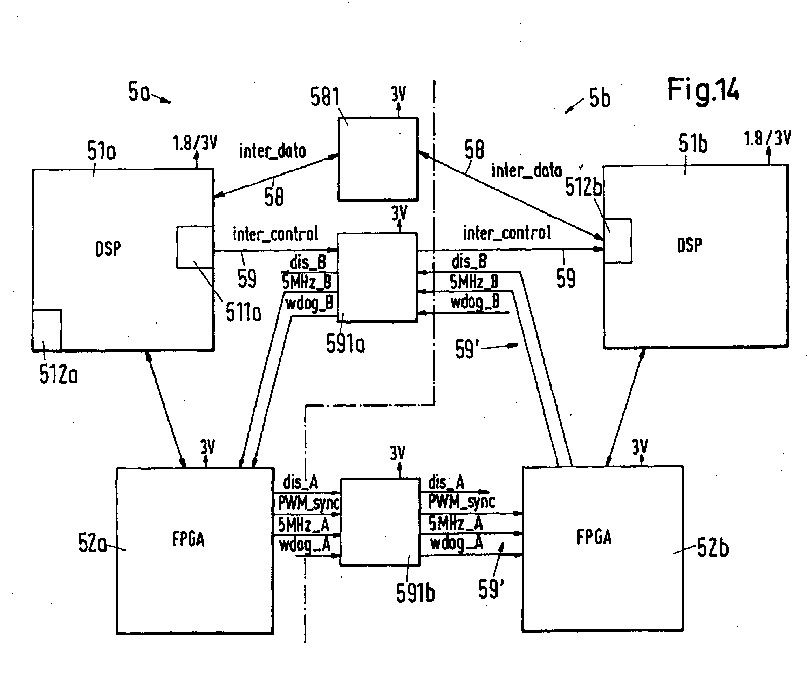

In Fig. 14 sind in einer schematischen Blockdarstellung die Interprozessorverbindungen 58,59 mit den Treibern oder Transceivem 581,591a,591b verdeutlicht. Die dort eingetragenen Signalbezeichnungen haben die bereits erläuterte Bedeutung, wobei der Buchstabe A bzw. B am Ende des Signalnamens das Kontrollsystem 5a bzw. 5b bezeichnet, auf welche sich das jeweilige Signal bezieht. So bezeichnet z. B wdog_A das Signal des Watchdogs 57a, der den DSP 51a des Masters 5a überwacht, während wdog_B das Signal des Watchdogs 57b bezeichnet, der den DSP 51b des Slave 5b überwacht.

Die Interprozessorverbindungen 58,59, über welche die beiden Kontrollsysteme 5a,5b miteinander kommunizieren und über welche sie sich gegenseitig überwachen und kontrolliern, umfassen eine Datenverbindung 58 (inter_data) sowie eine Kontrollverbindung 59 (inter_control). Damit die beiden Kontrollsysteme im fehlerfreien Normalbetrieb optimal zusammenarbeiten können, und damit sie auf Fehler angepasst reagieren können, benötigt jeder DSP 51a oder 51b einige Informationen von dem anderen DSP 51b oder 51a, beispielsweise Werte für die Position des Rotors, Werte für den Rotorwinkel, Informationen über den Status des jeweils anderen Kontrollsystems, Takt- und Synchronisationssignale, die Signale des Watchdogs 57a bzw. 57b, sowie die Möglichkeit, das jeweils andere Kontrollsystem zu deaktivieren. Die Daten (Werte für die Rotorposition, für den Rotorwinkel, usw.) werden dabei über die Interprozessorverbindung 58 ausgetauscht (inter_data), während die Kontrollsignale für die Kommunikation über die Interprozessorverbindung 59 ausgetauscht werden (inter_control). Diese Interprozessorverbindungen 58, 59 sind hauptsächlich durch Verbindungen (inter_control, inter_data) zwischen dem Daten/Adressbus 511a des DSP 51a des Masters 5a und einem HPI (Host Port Interface) 512b des DSP 51b des Slave 5b realisiert. Über diese Verbindungen 58 bzw. 59 kann der Master 5a alle benötigten Informationen aus dem Programm- und Daten-RAM des DSP 51b des Slave 5b auslesen. Zudem ist eine Verbindung 59' zwischen den FPGAs 52a,52b der beiden Kontrollsysteme 5a,5b vorgesehen, über welche die beiden FPGAs 52a,52b Kontrollsignale (dis_A,B; PWM_sync; 5MHZ_A,B; wdög_A,B) austauschen. Um eine einwandfreie Funktion auch im Falle des kompletten Ausfalls eines Kontrollsystems 5a oder 5b zu gewährleisten, ist in jeder physischen Interprozessorverbindung 58,59,59' jeweils einer der Treiber oder Transceiver 581,591a,591b vorgesehen, die beim kompletten Ausfall eines Kontrollsystems 5a oder 5b eine Blockierung des Daten/Adressbuses 511a oder des HPI 512b verhindern. Ansonsten könnte z. B. ein defekter Daten- oder Adressbuspin eines der beiden DSPs diese Busleitung auf einen Zustand (high oder low) reissen. Zudem ist in jeder physischen Interprozessorverbindung 58,59,59' ein seriell geschalteter Widerstand (nicht dargestellt) vorgesehen, welcher die Eingänge bzw. die Ausgänge der einzelnen Kontrollsysteme 5a,5b vor hohen Kurzschlussströmen schützt.

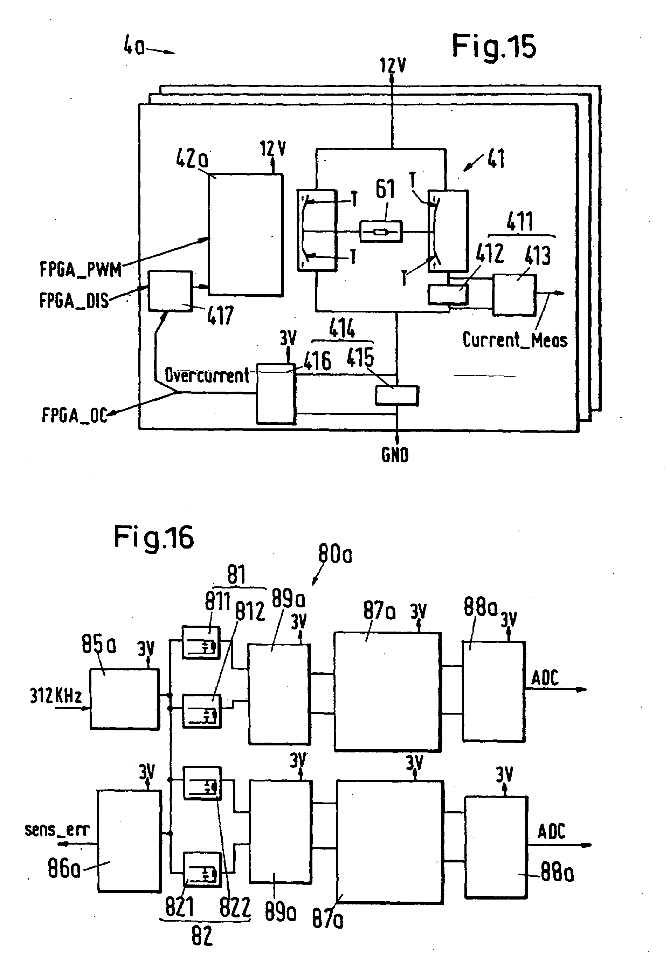

Fig. 15 zeigt schematisch den Aufbau des Leistungsteils 4a,4b, das, wie in Fig. 15 angedeutet, drei Subsysteme umfasst, nämlich eins für jeden Leistungsverstärker 41. In Fig. 15 ist nur eines dieser Subsyteme im Detail gezeigt. Das Subsystem des Leistungsteils 4a umfasst den Leistungsverstärker 41, der als H-Brücken-Schaltverstärker ausgebildet ist (siehe auch Fig. 6), und hier beispielsweise den Strang 61 (symbolisch angedeutet) der Antriebswicklung ansteuert. Die Schalttransistoren T sind beispielsweise n-Kanal Feldeffekttransistoren (FET) mit geringem Drain-Source-Widerstand R_{DSon}. Jeweils zwei FETs sind in einem gemeinsamen sehr kleinen Gehäuse angeordnet. Aufgrund des geringen Wertes von R_{DSon} ist keine zusätzliche Kühlung notwendig.

Ferner umfasst das Subsystem einen Treiber 42a zum Ansteuern der Schalttransistoren T. Der Treiber 42a wird mit den PWM-Signalen FPGA_PWM angesteuert, die von dem zugehörigen FPGA 52a generiert werden und ein Spannungsniveau von 3V haben. Der Treiber 42a transformiert diese Signale in 12V-Signale und steuert damit die oberen und die unteren Schalttransistoren T (gemäss der Darstellung) an. Der Treiber 42a umfasst ferner eine Schutzschaltung, die es verhindert, dass ein unterer und ein oberer FET gleichzeitig geschlossen sind. Der Treiber 42a kann durch ein Signal FPGA_DIS von dem FPGA 52a über einen Schaltkreis 417 deaktiviert werden. Im Fehlerfall können die FETs innerhalb etwa 100 Nanosekunden geöffnet werden.

Das Strommessgerät 411 zur Bestimmung des Phasenstroms umfasst einen Shunt-Widerstand 412 von beispielsweise 10-50 Milliohm sowie einen Operationsverstärker 413, der das Ausgangssignal des Shunt-Widerstands 412 verstärkt und als Signal Current_Meas an das DSP-Board 50a übermittelt. Der Shunt-Widerstand 412 ist in der H-Brücke zwischen der Source eines FET und dem negativen Betriebspotential, hier das Erdpotential der 12V-Gleichspannung, angeordnet und liefert ein Signal dessen Grösse linear von dem Phasenstrom abhängt.

Jedes Subsystem des Leistungsteils 4a umfasst zudem eine Überstromsicherung 414 mit einem weiteren Shunt-Widerstand 415 und einem weiteren Operationsverstärker 416. Der Ausgang diese Operationsverstärkers 416 ist mit dem Schaltkreis 417 verbunden, welcher den Treiber 42a abschalten kann. Der Shunt-Widerstand 415 der Überstromsicherung 414 ist zwischen der H-Brücke des Leistungsverstärkers 41 und dem negativen Betriebspotential, hier das Erdpotential der 12V-Gleichspannung, angeordnet. Die über den Shunt-Widerstand 415 abfallende Spannung wird in dem Operationsverstärker verstärkt und mit einem vorgebbaren Grenzwert verglichen, woraus ein digitales Ausgangssignal Overcurrent generiert wird. Im Falle eines unzulässig hohen Phasenstroms (Überstrom) wird das Signal FPGA_OC an das zugehörige FPGA 52a gesendet, welches den Treiber 42a abschaltet. Die Überstromsicherung 414 ist so ausgelegt, dass sie schnell genug ist, um einen kompletten Gleichstromkurzschluss zwischen den beiden Betriebspotentialen der 12V-Gleichspannung über die H-Brücke zu verhindern.

Jedes Kontrollsystem 5a,5b verfügt über ein eigenes, separates und komplettes Zwei-Achsen-Messsystem 80a,80b mit den Positionssensoren 81,82 bzw. 83,84 zur Bestimmung der radialen Position des Rotors 3. Fig. 16 zeigt das Blockschaltbild eines solchen Zwei-Achsen-Messsystems 80a mit den beiden Positionssensoren 81 und 82, die vorzugsweise jeweils die zwei Sensorelemente 811,812 bzw. 821,822 in 180°-Anordnung umfassen (siehe vorne, insbesondere Fig. 2 und Fig. 7). Mit jedem Zwei-Achsen-Messsystem 80a,80b sind jeweils zwei aktuelle Lagekoordinaten des Rotors bezüglich der X-Y-Ebene des Statorsystems bestimmbar, die im Folgenden einfach als x- und y-Komponente bezeichnet werden. Jedes Zwei-Achsen-Messsystem 80a,80b, dessen Achsen als x- und y-Achse bezeichnet werden, umfasst neben den Sensorelementen einen Anregungskreis 85a,85b, ein Monoflop 86a,86b, zwei Mixer 87a,87b, nämlich jeweils einen für jede Achse, sowie zwei Verstärker 88a,88b.

Der Anregungskreis 85a wird mit der 312 kHz-Frequenz gespeist, die von dem FPGA 52a generiert wird (siehe Fig. 13). Dieses Signal wird im Anregungskreis 85a verstärkt und den einzelnen Sensorelementen 811,812,821,822 zugeführt, die hier als Wirbelstomsensoren ausgestaltet sind. Das Ausgangssignal des Anregungskreises 85a wird von dem Monoflop 86a überwacht, welches ein Fehlersignal sens_err generiert und an das FPGA 52a übermittelt, sobald die Anregungsfrequenz nicht mehr vorhanden ist. Zur Auswertung wird das Ausgangssignal der Sensorelemente 811,812 für die x-Achse einem ersten Mixer 87a zugeführt und das Ausgangssignal der beiden Sensorelemente 821,822 für die y-Achse einem zweiten Mixer 87a. Die Mixer 87a, beispielsweise vom Typ PMB2331 der Firma Siemens, konvertieren die Ausgangssignale der Sensorelemente, die Wechselstrom-Signale sind, in Gleichstromsignale, welche von der aktuellen radialen Position des Rotors 3 abhängen. Diese Gleichstromsignale werden in den jeweils den Mixern 87a nachgeschalteten Verstärkern 88a verstärkt und dann an den ADC 54a (Fig. 12) übermittelt. Optional kann zwischen den Ausgängen des Positionssensors 81 und dem Eingang des zugehörigen Mixers 87a sowie zwischen den Ausgängen des Positionssensors 82 und dem Eingang des zugehörigen Mixers 87a noch jeweils ein Allpass-Filter 89a zwischengeschaltet sein, um die Phasenverschiebung zwischen dem Anregungssignal und dem Ausgangssignal der Sensorelemente abzugleichen.

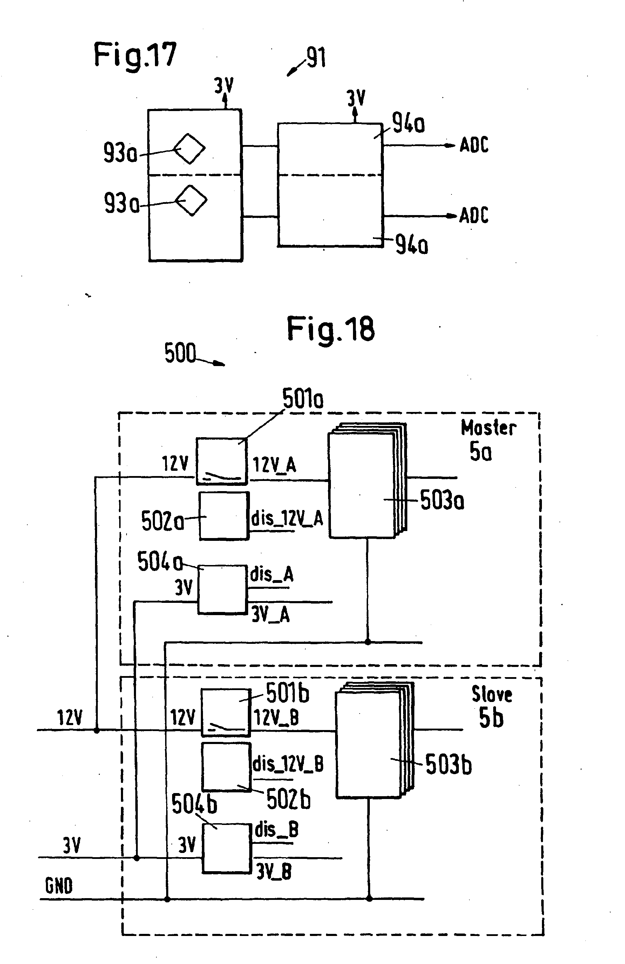

Fig. 17 zeigt ein Blockschaltbild des Feldsensors 91 oder 92, mit welchem der Rotorwinkel bestimmt wird. Jeder Feldsensor 91,92 umfasst zwei magnetoresistive Brücken 93a, beispielsweise vom Typ IMO LK15, die zwei um 90° phasenverschobene Signale, beispielsweise ein Sinus- und ein Cosinussignal, generieren, welche von dem Winkel und der Amplitude des magnetischen Feldes abhängen. Jeder Brücke 93a ist ein Operationsverstärker 94a nachgeschaltet, welcher das Ausgangssignal der jeweiligen Brücke 93a verstärkt und an den ADC 54a (siehe Fig. 13) übermittelt.

Fig. 18 zeigt in einem Blockschaltbild einen Versorgungskreis 500, welcher die Kontrollvorrichtung 5 mit Energie versorgt. Die Kontrollvorrichtung 5 benötigt zwei verschiedenen Gleichspannungen, nämlich im hier beschriebenen Ausführungsbeispiel 3 Volt und 12 Volt, wobei 12 Volt das positive Betriebspotential für die Leistungsverstärker 41 ist. Das negative Betriebspotential ist sowohl für die 3V-Versorgung als auch für die 12V-Versorgung jeweils das Erdpotential GND. Der Versorgungskreis 500 wird mit einer 3V- und mit einer 12V-Zuführung gespeist, die sich dann jeweils aufgabeln, um jedes der beidenKontrollsysteme 5a und 5b zu versorgen. In jedem der beiden Kontrollsysteme 5a,5b kann die 12V-Versorgung 12V_A bzw. 12V_B für dieses Kontrollsystem mittels eines Schalttransistors 501a,501b, beispielsweise vom Typ Si6954 der Firma Vishay, eingeschaltet oder abgeschaltet werden. Der Schalttransistor 501a,501b wird jeweils von einem Treiber 502a,502b angesteuert, bzw. geschaltet. Sobald dieser das Signal dis_12V_A bzw. dis_12V_B erhält, unterbricht er über den Schalttransistor 501a bzw. 501b die 12V-Versorgung des entsprechenden Kontrollsystems 5a bzw. 5b.

In der 3V-Versorgung 3V_A bzw. 3V_B jedes Kontrollsystems ist ein weiterer Treiber 504a,504b vorgesehen, der beim Anliegen des Signals dis_A bzw. dis_B die 3V-Versorgung dieses Kontrollsystems 5a oder 5b abschaltet. Das Signal dis_A bzw. dis_B zum Abschalten der jeweiligen 3V-Versorgung kann nur von dem FPGA 52a,52b (siehe Fig. 13) des jeweils anderen Kontrollsystems kommen, das heisst, nur das FPGA 52a des Kontrollsystems 5a kann die 3V-Versorgung des Kontrollsystems 5b unterbrechen, also das Signal dis_B generieren und nur das FPGA 52b des Kontrollsystems 5b kann die 3V-Versorgung des Kontrollsystems 5a unterbrechen, also das Signal dis_A generieren.

Der An/Aus-Pin für die 12V-Versorgung ist jeweils über einen Pull-Up-Widerstand 503a,503b mit der 3V-Versorgung des gleichen Kontrollsystems verbunden. Dieser gewährleistet, dass beim Abschalten der 3V-Versorgung für dieses Kontrollsystem automatisch die 12V-Versorgung für dieses Kontrollsystem folgt und auch abgeschaltet wird. Andererseits kann die 12V-Versorgung jedoch unabhängig von der 3V-Versorgung abgeschaltet werden, das heisst beim Deaktivieren der 12V-Versorgung in einem der beiden Kontrollsysteme 5a,5b bleibt die 3V-Versorgung dieses Kontrollsystems erhalten.

Der An/Aus-Pin für die 12V-Versorgung ist ferner über das zugehörige FPGA 52a,52b mit einer Überstromsicherung 414 (siehe Fig. 15) verbunden.

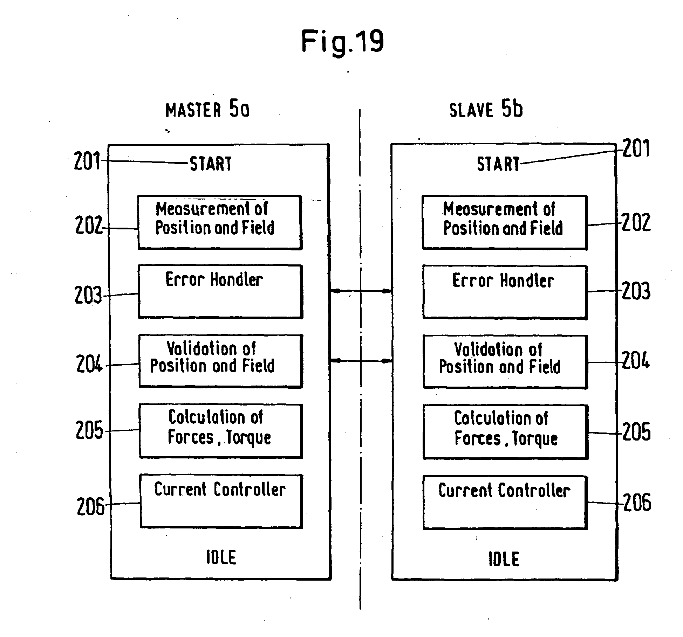

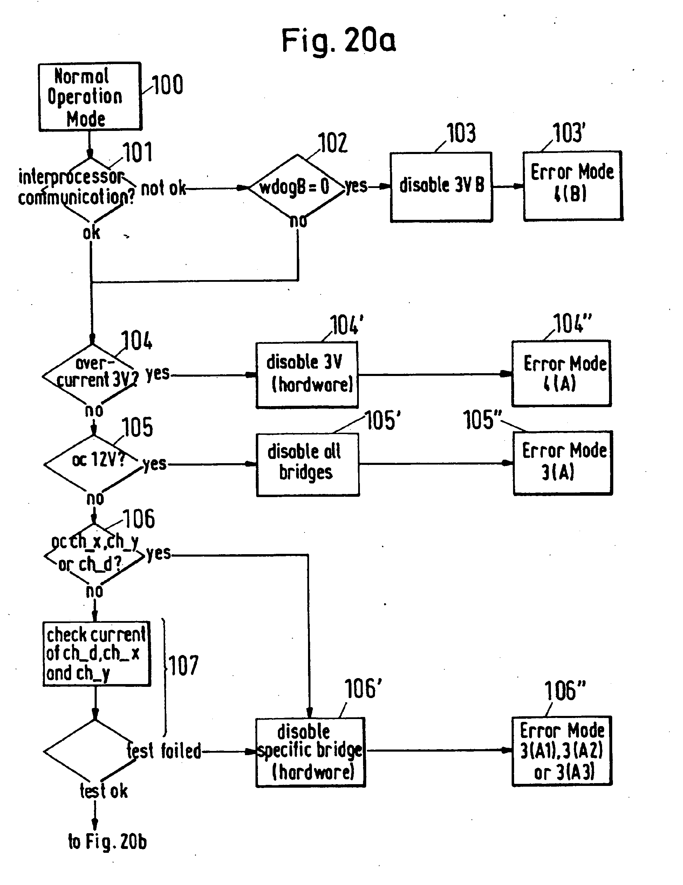

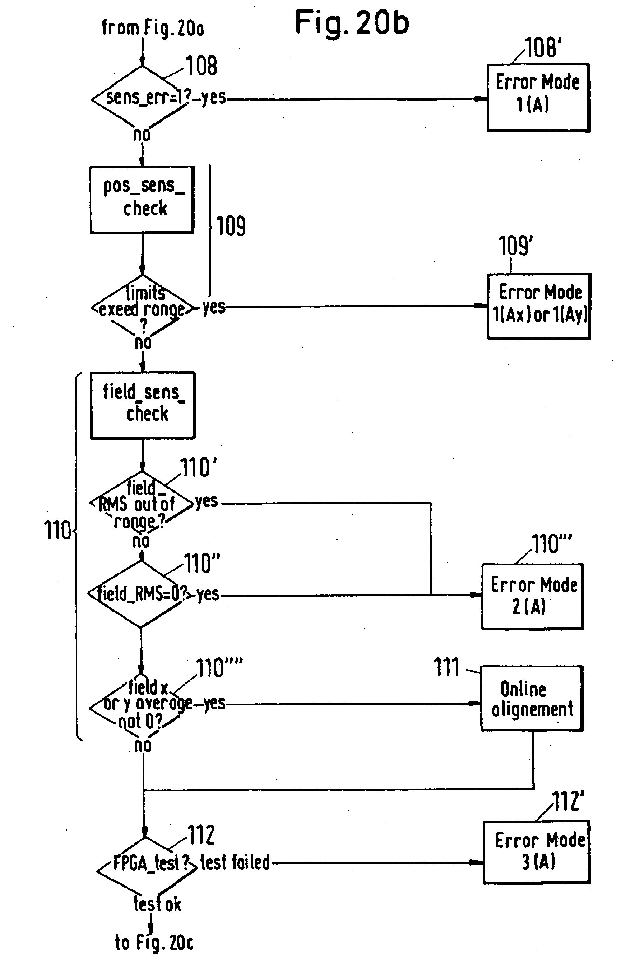

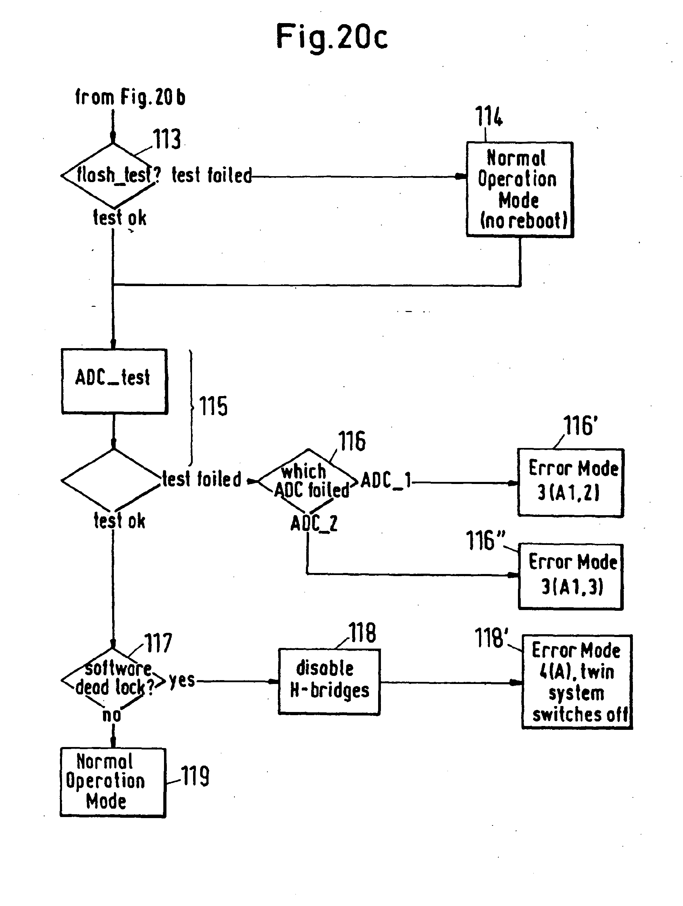

Fig. 19 zeigt ein Flussdiagramm eines Zyklus des prinzipellen Aufbaus der Software, das heisst des Programms, gemäss welchem die Kontrollvorrichtung 5 arbeitet. Die beiden Kontrollsysteme 5a,5b arbeiten parallel, wobei das prinzipelle Schema der Software für beide Kontrollsysteme 5a,5b identisch ist. Die Doppelpfeile in Fig. 19 zeigen symbolisch den Datenaustausch zwischen den beiden Kontrollsystemen 5a und 5b an. Im Schritt 201 (Start) werden die Zyklen für die beiden Kontrollsysteme 5a und 5b durch ein Interrupt-Signal initialisiert, welches von den PWM-Generatoren 521a,521b (Fig. 13) produziert wird. Die Startzeit liegt in einem Intervall, in welchem kein Schalten der Leistungsverstärker 41 erfolgt. Im Schritt 202 (Measurement of Position and Field) werden mittels der Positions- und Feldsensoren Werte für die Rotorposition und den Rotorwinkel ermittelt. Da kurz nach dem Start des Zyklus noch keine Störungen durch die FETs vorhanden sind, ist auch das Rauschen bei diesen Messungen sehr gering. Schritt 203 (Error handler) ist eine im Hintergrund laufende Aufgabe, bei welcher jedes Kontrollsystem 5a,5b das jeweils andere überprüft bzw. überwacht, indem beispielsweise abgefragt wird, ob das andere Kontrollsystem in einem vorgegebenen Zeitraum antwortet. Falls dem nicht so ist und das andere System nicht durch einen Reset geweckt werden kann, wird es komplett abgeschaltet (siehe auch Fig. 20 und die diesbezüglichen Erläuterungen). Im Schritt 204 (Validation of Position and Field) werden die Werte für die Position des Rotors und/oder die Werte für den Rotorwinkel, die jedes Kontrollsystem 5a,5b unabhängig von dem anderen Kontrollsysterm ermittelt hat, zwischen den beiden Kontrollsystemen 5a, 5b ausgetauscht. Der Master 5a überprüft die Werte des Slave 5b und entscheidet bei eventuell vorhandenen Abweichungen mit Hilfe von Fehlerroutinen, welche der Werte korrekt sind. Gegebenenfalls werden fehlerkorrigierte Werte bestimmt. Nachdem die korrekten oder fehlerkorrigierten Werte ermittelt sind, werden diese von beiden Kontrollsystemen übernommen, sodass beide Kontrollsysteme mit identischen Werten für die Position des Rotors und den Rotorwinkel arbeiten. Im Schritt 205 (Calculation of Forces, Torque) führt jedes Kontrollsystem 5a,5b die Regelroutinen für die Position und-die Geschwindigkeit des Rotors durch und ermittelt regelungstechnisch die benötigten Kräfte und Drehmomente, um den Rotor 3 im Stator 2 zentriert zu halten und um die Rotation des Rotors 3 mit der gewünschten Drehzahl aufrechtzuerhalten. Aus den so ermittelten Kräften und Drehmomenten werden die entsprechenden Phasenströme für die einzelnen Antriebs- und Steuerphasen ermittelt. Im Schritt 206 (Current Controller) werden die Leistungsverstärker derart angesteuert, dass sie die vorher ermittelten Phasenstöme in den Strang der jeweiligen Antriebs- oder Steuerphase einprägen.

Im normalen, das heisst fehlerfreien, Betriebszustand arbeiten die beiden Kontrollsysteme 5a,5b parallel (heisse Redundanz). Jedes Kontrollsystem 5a,5b führt seine eigene Positions- und Feldmessung durch, berechnet die benötigten Kräfte und das benötigte Drehmoment, und versorgt dementsprechend die Steuerphasen und die Antriebsphase mit dem jeweiligen Phasenstrom. Wie bereits erwähnt, tauschen die Kontrollsysteme 5a,5b die von ihnen jeweils ermittelten Werte für die Position des Rotors und für den Rotorwinkel miteinander aus, um sie aufeinander abzustimmen. Hierdurch ist es gewährleistet, das die beiden Kontrollsysteme 5a,5b nicht gegeneinander arbeiten.

Tritt nun ein Fehler in einem der beiden Kontrollsysteme 5a oder 5b auf, so sind verschiedene Fehlermodi vorgesehen, in denen der lagerlose Motor 1 betrieben werden kann, ohne dass die ordnungsgemässe magnetische Lagerung oder der ordnungsgemässe Antrieb des Rotors gefährdet ist. Im Folgenden werden nun einige Fehlermodi erläutert, wobei beispielhaft auf das Kontrollsystem 5a Bezug genommen wird. Es versteht sich, dass auch jeweils ein entsprechender Fehlermodus für den Fall vorgesehen ist, dass der gleiche Fehler im Kontrollsystem 5b auftritt. Der Buchstabe A in der Bezeichnung des Fehlermodus zeigt an, dass auf das Kontrollsystem 5a Bezug genommen wird.

Der Fehlermodus 1 (A) tritt auf, wenn in dem Zwei-Achsen-Messsystem 80a mit den , Positionssensoren 81,82 ein Fehler vorliegt. Dann wird dieses Messsystem komplett abgeschaltet und nur die vom Kontrollsystem 5b ermittelten Positionswerte werden für beide Kontrollsysteme 5a,5b herangezogen.

Die Fehlermodi 1(Ax) und (1Ay) treten auf, wenn nur eine Achse, nämlich die x- oder die y-Achse des Zwei-Achsen-Messsysystems 80a fehlerbehaftet ist. Dementsprechend wird auch nur die eine Achse x oder y abgeschaltet und der Positionswert, der mit der noch fehlerfreien Achse ermittelt wird, wird weiterhin zur Bestimmung der Position des Rotors herangezogen.

Der Fehlermodus 2(A) tritt ein, wenn der Feldsensor 91 des Kontrollsystems 5a fehlerhaft arbeitet und abgeschaltet wird. In diesem Modus erfolgt die Messung des Rotorwinkels nur noch mit dem Felddsensor 92 des Kontrollsystems 5b.

Der Fehlermodus 3(A) bedeutet, dass die 12V-Versorgung des Kontrollsystems 5a komplett abgeschaltet ist. In diesem Modus sind alle drei Leistungsverstärker 41 des Kontrollsystems 5a deaktiviert, der lagerlose Motor1 wird nur noch mit der Antriebsphase und den beiden Steuerphasen betrieben, die von dem Kontrollsystem 5b kontrolliert werden. Der DSP 51a und die Positionssensoren 81,82 sowie der Feldsensor 91 des Kontrollsystems 5a arbeiten in diesem Fehlermodus 3(A) noch.

Für den Fall, dass nur in einem oder in zwei Leistungsverstärker(n) 41 des Kontrollsystems 5a oder in einer oder zwei der Antriebs- und Steuerphasen, die vom Kontrollsystem 5a kontrolliert werden, ein Fehler auftritt, wird nur die jeweils fehlerbehaftete elektrische Phase abgeschaltet. Ein solcher Fehler kann zum Beispiel ein Leitungsbruch oder ein Kurzschluss in einer der Antriebs- oder Steuerspulen sein. Für diese Fälle sind die sechs Fehlermodi 3(A1), 3(A2), 3(A3), 3(A1,2), 3(A1,3), 3(A1,3), vorgesehen, wobei die Zahl oder die Zahlen in der Klammer jeweils angeben, welche der Phasen in dem jeweiligen Fehlermodus abgeschaltet ist. Dabei zeigt die Zahl 1 die Antriebsphase an und die Zahlen 2 und 3 die Steuerphasen. So bedeutet beispielsweise der Fehlermodus 3(A1,3), dass die Antriebsphase (1) und eine der beiden Steuerphasen (3) abgeschaltet ist und die andere Steuerphase (2) noch betrieben wird.

Im Fehlermodus 4(A) ist das gesamte Kontrollsystem 5a abgeschaltet. Der lagerlose Motor 1 wird nur noch mit dem Kontrollsystem 5b und den drei von diesem Kontrollsystem 5b kontrollierten Phasen betrieben.

Die Fig. 20a, 20b und 20c zeigen in ihrer Gesamtheit ein Flussdiagramm, das einen möglichen Ablauf einer Fehlererkennungsroutine veranschaulicht, wie sie im Schritt 203 des Software-Zyklus (siehe Fig. 19) durchgeführt wird. Die Reihenfolge der einzelnen Überprüfungen ist jedoch nur beispielhaft zu verstehen. Es wird wiederum auf das Kontrollsystem 5a Bezug genommen. Die Routine für das Kontrollsystem 5b sieht sinngemäss gleich aus.

Ausgehend vom normalen Betriebszustand 100 (Fig. 20a) werden im Schritt 101 die Interprozessorverbindungen 58,59 überprüft. Antwortet das Kontrollsystem 5b nicht, so wird im Schritt 102 der Watchdog 57b des Kontrollsystems 5b abgefragt. Ist dieser auf Null, wird im Schritt 103 die 3V-Versorgung des Kontrollsystems 5b abgeschaltet und im Schritt 103' der Fehlermodus 4(B) gewählt. Ist der Watchdog 57b nicht auf Null oder ist die Interprozessorverbindung in Ordnung, so wird in den Schritten 104 bis 106 auf Überstrom überprüft. Falls in der 3V-Versorgung (Schritt 104; overcurrent 3V?) ein Überstrom fliesst, wird im Schritt 104' die 3V-Versorgung abgeschaltet und im Schritt 104" der Fehlermodus 4(A) gewählt. Falls in der 12V-Versorgung ein Überstrom fliesst (Schritt 105; oc 12V?), werden im Schritt 105' alle drei Leistungsverstärker 41 abgeschaltet, und im Schritt 105" wird der Fehlermodus 3(A) gewählt. Falls in einer der drei Phasen ch_x, ch_y, ch_d ein Überstrom (oc) fliesst (Schritt 106), wird im Schritt 106' der entsprechende Leistungsverstärker 41 (H-Brücke) abgeschaltet und im Schritt 106" der zugehörige Fehlermodus gewählt. Im Schritt 107 werden die einzelnen Phasenströme auf Plausibilität überprüft (check current of ch_d, ch_x and ch_y). Falls dieser Test für eine der drei Phasen negativ ausfällt, wird mit Schritt 106' (siehe oben) fortgefahren. Im Schritt 108 (Fig. 20b) wird überprüft, ob ein Fehlersignal sens_err vom Zwei-Achsen-Messsystem 80a,80b vorliegt. Falls ja, wird im Schritt 108' in den Fehlermodus 1(A) gewechselt, falls nein wird im Schritt 109 überprüft, ob die Werte von den Positionssensoren einen vorgebbaren Bereich überschreiten (pos_sens_check; limits exceed range?). Falls ja, wird im Schritt 109' in den Fehlermodus 1(Ax) oder 1(Ay) gewechselt. Im Schritt 110 wird der Feldsensor 91 überprüft (field_sens_check). Falls der Root-Mean-Square-Wert (RMS) ausserhalb eines vorgebbaren Bereichs liegt (Schritt 110', field_RMS out of range?) oder gleich Null ist (Schritt 110", field_RMS=0?) wird im Schritt 110''' der Fehlermodus 2(A) gewählt. Im Schritt 110""" wird für beide Komponenten des Signals des Feldsensors überprüft, ob ihr Mittelwert von Null verschieden ist (field x or y average not 0?). Falls dem so ist, wird im Schritt 111 eine Kalibrierung (online alignment) des Feldsensors 91 durchgeführt. Im Schritt 112 wird das FPGA 52a überprüft (FPGA_test). Werden hierbei Fehler detektiert, so wird im Schritt 112' in den Fehlermodus 3(A) gewechselt. Im Schritt 113 (Fig. 20c) wird der Flash-Speicher 53a getestet (flash_test). Reagiert dieser nicht, bzw. lässt er sich nicht aufwecken, so wird im Schritt 114 registriert, dass das Kontrollsystem 5a nicht neu gestartet werden kann, aber der normale Betriebszustand beibehalten werden kann. Im Schritt 115 werden die ADCs 54a überprüft (ADC_test). Falls bei diesem Test Fehler detektiert werden, wird im Schritt 116 überprüft, welcher der beiden ADCs 54a fehlerbehaftet ist und dementsprechend im Schritt 116' oder 116" in den Fehlermodus 3(A1,2) oder 3(A1,3) gewechselt. Im Schritt 117 wird schliesslich noch die Software überprüft (software dead lock). Falls einzelne Software-Module länger dauern als vorgesehen (z. B. bei einem Aufhängen der Software), wird der Watchdog nicht mehr angesprochen, was zu einem Reset führt. Das andere Kontrollsystem 5b überwacht den Reset und stellt nach mehrmaligem Auftreten des Fehlers das Kontrollsystem 5a ab. Falls bei diesem Test ein Fehler auftritt, werden im Schritt 118 alle Leistungsverstärker (H-Brücken) des Kontrollsystems 5a abgeschaltet, und es wird im Schritt 118' in den Fehlermodus 4(A) gewechselt, indem das andere Kontrollsystem 5b das gesamte Kontrollsystem 5a deaktiviert. Wenn alle Überprüfungen zu einem positiven, das heisst fehlerfreien, Ergebnis geführt haben, wird der normale Betriebszustand fortgesetzt (Schritt 119).

Durch die verschiedenen Fehlermodi ist es möglich, sehr flexibel auf eine Vielzahl von verschiedenartigen Fehlern optimal angepasst zu reagieren. Im Folgenden wird nun in einer nicht abschliessenden Aufzählung und in tabellarischer Form angegeben, wie verschiedene Fehler erkannt und behoben werden. Dabei wird wiederum nur auf das Kontrollsystem 5a Bezug genommen. Natürlich gelten die Erläuterungen in sinngemäss gleicher Weise auch für das andere Kontrollsystem 5b. In der ersten Spalte der Tabelle ist jeweils eine Laufnummer angegeben, in der zweiten Spalte wird der Fehler spezifiziert, in der dritten Spalte wird angegeben, wie bzw. mit welchen Elementen der Fehler erkennbar ist, und in der vierten Spalte wird angegeben, wie auf den Fehler reagiert wird, sowie gegebenenfalls der Fehlermodus, der verwendet wird. Fehlermodus wird dabei mit FM abgekürzt. Die Abkürzung IPV steht für Interprozessorverbindung.

| **1** | **Zwei-Achsen-Messsystem 80a** | | |
|---|---|---|---|
| *1.1* | *Keine Anregung* | | |
| 1.1.1 | Kein Takt von DSP 51a | DSP tot; keine IPV; Watchdog | Kontrollsystem 5a abschalten; FM 4(A) |
| 1.1.2 | Kein Takt von FPGA 52a | FPGA tot; DSP des Kontrollsystems 5b | Siehe 1.1.1 |
| 1.1.3 | Kabelbruch | Sens_err Signal Monoflop 86a | Messsystem abschalten; FM 1(A) |
| 1.1.4 | Schaden im Anregungskreis 85a | Siehe 1.1.3 | Siehe 1.1.3 |
| *1.2* | *Positionssensor 81,82* / *Mixer 87a* | | |
| 1.2.1 | Ausfall Mixer 87a | Sensortest in DSP | Abschalten der Achse FM 1 (Ax) oder 1 (Ay) |
| 1.2.2 | Ausfall Verstärker 88a Sensortest in DSP | | Siehe 1.2.1 |
| 1.2.3 | Ausfall Sensor Sens_err Signal Monoflop (Kurzschluss) 86a | | Siehe 1.1.3 |
| 1.2.4 | Ausfall Sensor (offen) Sensortest in DSP | | Siehe 1.2.1 |
| *1.3* | *Verstärkung* / *Offset* | | |
| 1.3.1 | Alterung Widerstände | Sensortest in DSP | Siehe 1.2.1 |
| 1.3.2 | Temperatureffekte | Sensortest in DSP | Siehe 1.2.1 |
| *1.4* | *Kabelbruch* | | |
| 1.4.1 | Alle Kabel | Sens_err Signal geht hoch | Siehe 1.2.1 |
| 1.4.2 | Ein Kabel | Sensortest in DSP | Siehe 1.2.1 |
| *1.5* | *Drift der Anregungsfrequenz* | | |
| 1.5:1 | Alterung Kondensatoren | Sensortest in DSP | Siehe 1.2.1 |
| 1.5.2 | Temperatureffekte | Sensortest in DSP | Siehe 1.2.1 |
| | | | |
| **2** | **Feldsensor 91** | | |
| *2.1* | *Keine Anregung* | | |
| 2.1.1 | Keine 3V-Versorgung (Kabelbruch) | Signal an Grenze | Feldsensor abschalten; FM 2(A) |
| 2.1.2 | Weder 3V noch GND (Kabelbrüche) | Signal schwebt; RMS des Signals nicht korrekt | Siehe 2.1.1 |
| *2.2* | *Bruch Sensorchip* | | |
| 2.2.1 | Kurzschluss interner Widerstände | Signal an Grenze | Siehe 2.1.1 |
| 2.2.2 | Interne Widerstände offen (leiten nicht) | Signal schwebt; RMS des Signals nicht korrekt | Siehe 2.1.1 |
| *2.3* | *Drift* | | |
| 2.3.1 | Offset-Drift (Alterung) | Mittelwert ungleich Null | Kalibrierung online (kein Fehler) |
| 2.3.2 | Verstärkung(Alterung) | RMS des Signals nicht korrekt | Siehe 2.3.1 |
| *2.4* | *Ausfall Operationsverstärker* | *94a* | |
| 2.4.1 | Kurzschluss Ausgang oder Widerstände | Signal an Grenze | Siehe 2.1.1 |
| 2.4.2 | Offener Ausgang | Signal schwebt; RMS des Signals nicht korrekt | Siehe 2.1.1 |
| 2.4.3 | Drift Offset | Siehe 2.3.1 | Siehe 2.3.1 |
| 2.4.4 | Drift Verstärkung | Siehe 2.3.1 | Siehe 2.3.1 |
| *2.5* | *Kabelbrüche* | | |
| 2.5.1 | Alle Kabel | Siehe 2.1.2 | Siehe 2.1.2 |
| 2.5.1 | Einzelne Kabel | Signal an Grenze, konstant oder schwebend | Siehe 2.1.1 |
| **3** | **Antrieb** | | |
| *3.1* | *Strang der Antriebswicklung* | | |
| 3.1.1 | Totaler Kurzschluss | Überstrom (keine Last) | Zugehörige H-Brücke abschalten, FM 3(A1) |
| 3.1.2 | Partieller Kurzschluss Induktanz noch gross | Kein Fehler | Keine |
| 3.1.3 | Partieller Kurzschluss Induktanz klein | Siehe 3.1.1 | Siehe 3.1.1 |
| 3.1.4 | Strang nicht leitend | Kein Strom | Leistungsteil 4a abschalten, FM 3(A) |
| *3.2* | *Versorgung* | | |
| 3.2.1 | Kurzschluss zu GND | Überstrom 12V-Versorg. | Siehe 3.1.4 |
| 3.2.2 | Kurzschluss zu 12V | Siehe 3.2.1 | Siehe 3.2.1 |
| | | | |
| **4** | **Lagerung** | | |
| *4.1* | *Stränge der Steuerwicklung* | | |
| 4.1.1 | Totaler Kurzschluss | Überstrom (keine Last) | Zugehörige H-Brücke abschalten, FM 3(A1) |
| 4.1.2 | Partieller Kurzschluss Induktanz noch gross | Kein Fehler | Keine |
| 4.1.3 | Partieller Kurzschluss Induktanz klein | Siehe 4.1.1 | Siehe 4.1.1 |
| 4.1.4 | Strang nicht leitend | Kein Strom | Siehe 4.1.1 |
| *4.2* | *Versorgung* | | |
| 4.2.1 | Kurzschluss zu GND | Überstrom 12V-Versorg. | Leistungsteil 4a abschalten, FM 3(A) |
| 4.2.2 | Kurzschluss zu 12V | Siehe 4.2.1 | Siehe 4.2.1 |
| | | | |
| **5** | **Leistungsteil 4a** | | |
| *5.1* | *Überstrom* | | |
| 5.1.1 | Kurzschluss eines FET | Überstromsicherung 414 (Hardware) | Zugehörige H-Brücke abschalten, FM 3(A1), 3(A2) oder 3(A3) |
| 5.1.2 | Kurzschluss Strang | Siehe 5.1.1 | Siehe 5.1.1 |
| 5.1.3 | Ausfall Treiber 42a | Siehe 5.1.1 | Siehe 5.1.1 |
| 5.1.4 | Ausfall Überstromsicherung | Siehe 5.1.1 | Siehe 5.1.1 |
| 5.1.5 | PWM-Signal blockiert | Überstrom-Detektion (Software) | Leistungsteil 4a abschalten (12V-Vers.) FM 3(A) |
| *5.2* | *Kein Strom* | | |
| 5.2.1 | PWM-Signal blockiert | Summe der RMS ist Null, während Spannung ungleich Null ist | Zugehörige H-Brücke abschalten FM 3(A1), 3(A2) oder 3(A3) |
| 5.2.2 | FET offen (nicht leitend) | Siehe 5.2.1 | Siehe 5.2.1 |
| 5.2.3 | Ausfall Strommessgerät | Siehe 5.2.1 | Siehe 5.2.1 |
| 5.2.4 | Ausfall Treiber 42a FET | Siehe 5.2.1 | Siehe 5.2.1 |
| *5.3* | *Kondensator in der Gleichstromverbindung* | | |
| 5.3.1 | Kurzschluss Kondensator | Überstrom 12V Eingang | 12V abschalten; FM 3(A) |
| 5.3.2 | Kondensator offen | Kein Fehler | Keine |
| | | | |
| **6** | **DSP-Board 50a** | | |
| *6.1* | *DSP 51a* | | |
| 6.1.1 | Ausfall DSP | Keine IPV, Watchdog 57a | Kontrollsystem 5a abschalten FM 4(A) |
| 6.1.2 | Keine 3V-Versorgung | Keine IPV, Watchdog 57a | Siehe 6.1.1 |
| 6.1.3 | Keine 1.8V-Versorgung | Keine IPV, Watchdog 57a | Siehe 6.1.1 |
| 6.1.4 | Software dead lock | Watchdog 57a, FPGA Watchdog | Leistungsteil 4a und 12V sofort abschalten; wie 6.1.1 |
| *6. 2* | *Interprozessor-Kommunikation* | | |
| 6.2.1 | Ausfall FPGA 52a | Keine IPV | Siehe 6.1.1 |
| 6.2.2 | Ausfall Treiber 581,591a | Keine IPV | Siehe 6.1.1 |
| *6.3* | *ADC 54a* | | |
| 6.3.1 | Ausfall ADC, unmögliche Werte | ADC-Test negativ | Entsprechend Phasen abschalten FM 3(A1,2) oder 3A(1,3) |
| 6.3.2 | Ausfall ADC, mögliche Werte | Siehe 6.3.1 | Siehe 6.3.1 |
| *6.4* | *FPGA 52a* | | |
| 6.4.1 | Kein Takt von DSP | Ausfall DSP: FPGA_watchdog (getaktet von Kontrollsystem 5b) | Leistungsteil 4a abschalten, FM 4(A) |
| | | Ausfall Taktausgang: FPGA_test zählt nicht | Leistungsteil 4a abschalten, FM 4(A) |
| 6.4.2 | Keine 3V-Versorgung | Siehe 6.1.1 | Siehe 6.1.1 |
| 6.4.3 | PWM Ausgänge blockiert | Überstrom oder kein Strom (Software) | Leistungsteil 4a abschalten, FM 4(A) |
| 6.4.4 | Ausgänge power_enable blockiert | Betrieb noch möglich | Keine |
| *6.5* | *Flash-Speicher 53a* | | |
| 6.5.1 | Ausfall Flash-Speicher | Kein Neustart, check-Summe stimmt nicht | Keine Aktion während Betrieb |
| 6.5.2 | Ausfall Flash Brennfunktion (buming) | | Keine Aktion während Betrieb |
| *6.6* | *Peripherie* | | |
| 6.6.1 | Kein Taktsignal | DSP arbeitet nicht, keine IPV | Kontrollsystem 5a abschalten, FM 4(A) |
| 6.6.2 | Ausfall Watchdog 57a | Reset DSP während normalem Betrieb | Kontrollsystem 5a abschalten, FM 4(A) |
| 6.6.3 | Linearregler 56a (1.8V) | Siehe 6.1.3 | Siehe 6.1.3 |
| | | | |
| **7** | **Versorgungskreis 500** | | |
| *7.1* | *3V-Versorgung* | | |
| 7.1.1 | Kurzschluss im 3V-System | Überstrom 3V-Versorgung | Kontrollsystem 5a abschalten (3V und 12V); FM 4(A) |
| 7.1.2 | Offener 3V Schalter 504a | Keine IPV, Watchdog 57a | Anderes Kontrollsystem übernimmt; FM 4(A) |
| *7.2* | *12V-Versorgung* | | |
| 7.2.1 | Kurzschluss im 12V-System | Überstrom 12V-Versorg. | Abschalten 12V-Versorgung FM 3(A) |
| 7.2.2 | 12V-Versorgung offen (Unterbrechung) | Kein Strom in den H-Brücken | Anderer Leistungsteil 4b übernimmt, Messsysteme arbeiten noch; FM 3(A) |

In der Tabelle bedeutet "Signal an Grenze", dass das zugehörige Signal seinen maximal oder minimal möglichen Wert annimmt. Der "Sensortest in DSP" umfasst beispielsweise die im Schritt 109 (siehe Fig. 20) durchgeführte Überprüfung, bei welcher überprüft wird, ob die Werte der Positionssensoren innerhalb vorgegebener Grenzen liegen. Der Sensortest kann aber auch noch andere Massnahmen umfassen: Da jeder der beiden Positionssensoren 81,82 des Kontrollsystems 5a jeweils zwei Sensorelemente 811,812 bzw. 821,822 in 180°-Anordnung umfasst (siehe vorne), können durch Differenzbildung systematische Fehler (Offset, Temperaturerffekte, Alterungseffekte) erkannt und kompensiert werden (Kalibrierung). Auch ist es möglich, die Signale der einzelnen Positionssensoren bzw. Sensorelemente auf gegenseitige Kompatibilität zu prüfen, indem beispielsweise aus jeweils zwei Signalen ein drittes berechnet und mit dem entsprechenden gemessenen Signal verglichen wird (Zwei-aus-Drei-Test). Auf diese Weise kann ein fehlerhaftes Sensorelement identifiziert werden. Ferner ist es zur Fehlererkennung möglich, die Positionswerte, die von den beiden Kontrollsystemen 5a und 5b unabhängig voneinander ermittelt wurden, miteinander zu vergleichen. Da von beiden Kontrollsystemen 5a,5b insgesamt vier unabhängige Positionswerte für die beiden Koordinaten des Rotors 3 in der X-Y-Ebene vorliegen, kann anhand dieses überbestimmten Gleichungssystems (vier Gleichungen, zwei Unbekannte) festgestellt werden, ob einer und gegebenenfalls welcher der Positionswerte falsch ist. Gleiches gilt auch für die Werte der beiden unabhängigen Feldsensoren. Auch diese bilden ein Gleichungssystem mit vier Gleichungen und zwei Unbekannten.

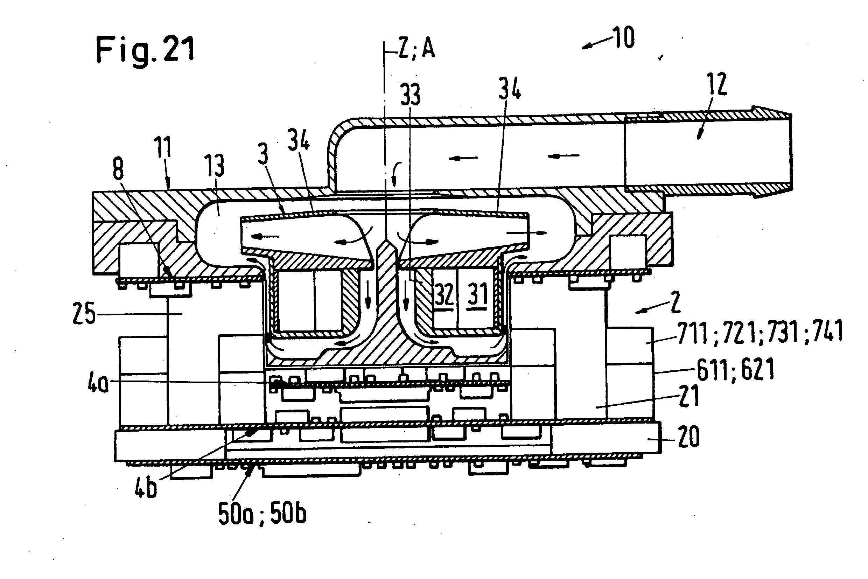

Durch die Erfindung wird ferner eine Blutpumpe vorgeschlagen, die einen erfindungsgemässen Drehantrieb umfasst, und die insbesondere auch zur Implantation in einen Körper geeignet ist. Fig. 21 zeigt in einer stark schematisierten Darstellung einen Querschnitt durch ein bevorzugtes Ausführungsbeispiel einer erfindungsgemässen Blutpumpe, die gesamthaft mit dem Bezugszeichen 10 bezeichnet ist, und als Zentrifugalpumpe ausgebildet ist.

Der erfindungsgemässe Drehantrieb mit dem Stator 2 und dem Rotor 3 ist vorzugsweise als Tempelmotor (siehe auch Fig. 9) mit acht jeweils L-förmigen Statorzähnen ausgebildet, von denen in Fig. 21 nur zwei, nämlich die mit 21 und 25 bezeichneten, sichtbar sind. Die Statorzähne 21,25 sind über den ringförmigen Eisenrückschluss 20 magnetisch miteinander gekoppelt. Um die langen Schenkel der Statorzähne 21,25 sind, wie bereits vorne beschrieben, die diskreten Antriebsspulen 611,621 und die diskreten Steuerspulen 711,721,731,741 der Antriebswicklung bzw. der Steuerwicklung angeordnet. Dabei sind wie in Fig. 2 gezeigt vier konzentrierte Antriebsspulen 611,621 vorgesehen, die wie in Fig. 5 dargestellt zu den beiden Strängen 61,62 der Antriebswicklung zusammengeschaltet sind. Bei dem Ausführungsbeispiel gemäss Fig. 21 sind entweder acht konzentrierte Steuerspulen 711,712,731,741 vorgesehen nämlich auf jedem Statorzahn eine, analog wie dies in Fig. 2 gezeigt ist, oder sechszehn konzentrierte Steuerspulen 711,712,713,714, nämlich auf jedem Statorzahn zwei, analog wie dies in Fig. 7 gezeigt ist. Im letzteren Fall sind bevorzugt die beiden auf dem gleichen Statorzahn vorgesehen Steuerspulen jeweils aufeinander gewickelt, so wie dies vorne erläutert wurde. Die acht bzw. sechszehn Steuerspulen sind in analoger Weise wie es in Fig. 4 bzw. in Fig. 8 dargestellt ist, zu den vier Strängen 71-74 der Steuerwicklung zusammengeschaltet. Der Drehantrieb der Blutpumpe 10 hat also bei diesem Ausführungsbeispiel zwei Antriebsphasen und vier Steuerphasen.

Der permanentmagnetisch erregte Rotor 3 mit dem permanentmagnetischen Ring 31 und dem Eisenrückschluss 32 weist ferner eine Ummantelung 33, vorzugsweise aus Kunststoff, auf sowie mehrere Flügel 34 zum Fördern des Bluts, das heisst der Rotor 3 des Drehantriebs dient auch als Pumpenrotor. Er bildet also einen sogenannten Integralrotor. Der Rotor 3 ist im Betrieb im Stator 2 magnetisch gelagert und wird vom Stator 2 angetrieben, sodass er um die Drehachse A rotiert.

Der Rotor 3 ist in einem Pumpengehäuse 11 angeordnet, welches einen Einlass 12 für das zu fördernde Blut aufweist. Im Betrieb strömt das Blut, symbolisch angedeutet durch die Pfeile ohne Bezugszeichen, durch den Einlass 12 und dann aus der axialen Richtung auf den Rotor 3 zu, wo es von den Flügeln 34 in die radiale Richtung umgelenkt und in einen Auslasskanal 13 gefördert wird, welcher in einen in Fig. 21 nicht erkennbaren Auslass mündet. Wie dies die Pfeile andeuten, strömt ein Teil des Bluts in axialer Richtung durch den Rotor 3 hindurch, sodass es darstellungsgemäss unterhalb des Rotors 3 eine hydrodynamische Lagerung bewirkt, welche die magnetische Lagerung unterstützt.

Der Betrieb der Blutpumpe 10 wird besonders bevorzugt mit der vorangehend ausführlich beschriebenen Kontrollvorrichtung 5, welche die beiden separaten Kontrollsysteme 5a,5b umfasst, geregelt und kontrolliert. Im Hinblick auf eine möglichst kompakte Ausgestaltung sind die Kontrollsysteme 5a,5b jeweils in Form von mehreren Elektronikprints oder Chips realisiert, die zumindest teilweise im Innern des Drehantriebs angeordnet sind. Wie dies Fig. 21 zeigt, sind vorzugsweise diejenigen Elektronikprints, welche die Leistungsteile 4a,4b mit den Leistungsverstärkern 41 tragen, im Innern des Drehantriebs in dem von den Statorzähnen 21,25 umgebenen Raum und darstellungsgemäss unterhalb des Rotors angeordnet. Neben dem Vorteil einer optimierten Raumausnutzung und damit einer sehr kompakten Bauweise, hat diese Massnahme den Vorteil, dass die einzelnen Leistungsverstärker 41 jeweils unmittelbar, das heisst ohne Kabel, mit den zugehörigen Antriebs- bzw. Steuerspulen verbindbar sind. Durch diese Massnahme ist die Anzahl der Kabel bzw. Kabeladern, die vom Drehantrieb weg nach aussen geführt werden müssen, so gering wie möglich. Dies bedeutet eine erhebliche Steigerung der Betriebssicherheit, weil Kabelverbindungen erfahrungsgemäss die höchste Ausfallrate haben. Durch die geringe Anzahl der Kabelverbindungen ist es zudem möglich, alle Kabelverbindungen redundant auszugestalten, um auch hier eine Fehlertoleranz zu realisiem.

Die DSP-Boards 50a,50b sind vorzugsweise darstellungsgemäss unmittelbar unterhalb des Stators 2 angeordnet.

Eine weitere vorteilhafte Massnahme besteht darin, ein Auswertemodul 8 für die Positionssensoren 81,82 bzw. 83,84 vorzusehen, das als ringförmiger Elektronikprint ausgestaltet ist und derart auf den kürzeren Schenkeln der Statorzähne 21,25 angeordnet ist, dass sich die auf dem Elektronikprint vorgesehenen Bauteile, insbesondere die Positionssensoren, in dem freien Raum zwischen den Statorzähnen 21,25 befinden. Wie dies Fig. 21 zeigt, ist das Auswertemodul 8 zwischen den kurzen Schenkeln der Statorzähnen 21,25 und dem Pumpengehäuse 11 angeordnet. Das Auswertemodul 8 ist beispielsweise wie in Fig. 16 gezeigt aufgebaut bzw. bestückt und umfasst vorzugsweise beide Zwei-Achsen-Messsystem 80a,80b.

Eine weitere vorteilhafte Massnahme besteht darin, die einzelnen Elektronikprints durch flexible Prints (Flexprints) miteinander zu verbinden. Die festen, das heisst im wesentlichen starren, Elektronikprints bilden dann zusammen mit den flexiblen Verbindungsprints einen sogenannten Rigid-Flex-Verbund, welcher als Einheit gefertigt, bestückt und geprüft werden kann.

Die Blutpumpe 10 zeichnet sich aus durch ihre extrem hohe Betriebssicherheit, ihre Fehlertoleranz, mit der sie auf eine Vielzahl verschiedenartiger Fehler reagieren kann, ohne dass dadurch der ordnungsgemässe Betrieb sowohl bezüglich des Antriebs des Rotors 3 als auch seiner magnetischen Lagerung gefährdert wird, ihre sehr kompakte, platzsparende Ausgestaltung, die optimierte Ausnutzung des zur Verfügung stehenden Raums und den vergleichsweise geringen Energiebedarf, wobei sie gleichzeitig eine hohe Leistungsfähigkeit aufweist.

## Patentansprüche

1. Fehlertoleranter elektrischer Drehantrieb, ausgestaltet als lagerloser Motor, mit einem magnetisch gelagerten Rotor (3) und einem Stator (2), der eine mindestens zwei Stränge (61,62) aufweisende Antriebswicklung (6) zum Erzeugen eines magnetischen Antriebsfelds umfasst, welches ein Drehmoment auf den Rotor (3) bewirkt, und eine mindestens drei Stränge (71,72,73,74) aufweisende Steuerwicklung (7) zum Erzeugen eines magnetischen Steuerfelds, mit welchem die Position des Rotors (3) bezüglich des Stators (2) regelbar ist, wobei jeder Strang (61,62) der Antriebswicklung (6) zu einer anderen elektrischen Antriebsphase gehört, und jeder Strang (71-74) der Steuerwicklung (7) zu einer anderen elektrischen Steuerphase, sowie mit einer Stelleinrichtung (4), die jeden Strang (61,62) der Antriebswicklung (6) und jeden Strang (71-74) der Steuerwicklung (7) jeweils mit einem Phasenstrom oder einer Phasenspannung als Stellgrösse versorgt, **dadurch gekennzeichnet, dass** eine Kontrollvorrichtung (5) vorgesehen ist, die ein unabhängiges Ansteuern jeder einzelnen Antriebsphase und jeder einzelnen Steuerphase ermöglicht, sodass mit der Stelleinrichtung (4) die Stellgrösse für jeden Strang (61,62) der Antriebswicklung (6) und für jeden Strang (71-74) der Steuerwicklung (7) unabhängig von den Stellgrössen für die anderen Stränge regelbar ist, wobei die Stelleinrichtung (4) für jeden Strang (61,62) der Antriebswicklung (6) und für jeden Strang (71-74) der Steuerwicklung (7) einen separaten bipolaren Leistungsverstärker (41) umfasst.

2. Drehantrieb nach Anspruch 1 oder 2, bei welchem die Antriebswicklung (6) eine Polpaarzahl p aufweist und die Steuerwicklung (7) eine Polpaarzahl p±1.

3. Drehantrieb nach einem der vorangehenden Ansprüche, ausgestaltet als Tempelmotor, bei welchem der Stator (2) mehrere durch einen Rückschluss (20) verbundene Statorzähne (21-28) aufweist, die jeweils L-förmig ausgebildet sind, wobei sich der längere Schenkel (SL) in der axialen Richtung erstreckt, welche durch die Soll-Drehachse (A) des Rotors (3) festgelegt ist, und der kürzere Schenkel (SK) radial nach innen verläuft.

4. Drehantrieb nach einem der vorangehenden Ansprüche mit genau drei Antriebsphasen und mit genau drei Steuerphasen.

5. Drehantrieb nach einem der Ansprüche 1-4 mit genau zwei Antriebsphasen und mit genau vier Steuerphasen.

6. Drehantrieb nach einem der vorangehenden Ansprüche, bei welchem der Stator genau acht Statorzähne (21-28) aufweist, zwischen denen der Rotor (3) gelagert ist.

7. Drehantrieb nach Anspruch 6 mit zwei Antriebsphasen, wobei die Antriebswicklung (6) vier konzentrierte Antriebsspulen (611,621) umfasst, und jede Antriebsspule (611,621) um zwei unmittelbar benachbarte Statorzähne (21-28) gewickelt ist, wobei jeweils zwei, sich diametral gegenüberliegende Antriebsspulen (611;621) einen Strang (61;62) der Antriebswicklung (6) bilden und somit zur gleichen Antriebsphase gehören.

8. Drehantrieb nach einem der vorangehenden Ansprüche, bei welchem die Steuerwicklung (7) mehrere konzentrierte Steuerspulen (711,721,731,741) umfasst, von denen jede um einen anderen Statorzahn gewickelt ist.

9. Drehantrieb nach einem der vorangehenden Ansprüche, bei welchem die Steuerwicklung (7) mehrere konzentrierte Steuerspulen (711,721,731,741) umfasst, wobei jeweils die um einen ersten Statorzahn gewickelte Steuerspule und die um den in Umfangsrichtung gesehen übernächsten Statorzahn gewickelte Steuerspule zur gleichen Steuerphase gehören.

10. Drehantrieb nach einem der vorangehenden Ansprüche mit vier Steuerphasen, bei welchem die Steuerwicklung (7) acht konzentrierte Steuerspulen (711,721,731,741) umfasst, wobei jeweils zwei Steuerspulen (711;721;731;741) einen Strang (71;72;73;74) der Steuerwicklung (7) bilden und somit zur gleichen Steuerphase gehören.

11. Drehantrieb nach einem der vorangehenden Ansprüche mit vier Steuerphasen, bei welchem die Steuerwicklung (7) sechzehn konzentrierte Steuerspulen (711,721,731,741) umfasst, wobei auf jedem Statorzahn jeweils zwei Steuerspulen (711,721,731,741) vorgesehen sind, und wobei jeweils vier Steuerspulen (711;721;731;741), die um vier verschiedenen Statorzähne gewickelt sind, derart zu einem Strang (71;72;73;74) der Steuerwicklung (7) zusammengeschaltet sind, dass, in Umfangsrichtung gesehen, jeweils die um unmittelbar benachbarte Statorzähne gewickelten Steuerspulen zu verschiedenen Strängen der Steuerwicklung gehören.

12. Drehantrieb nach Anspruch 11, bei welchem jeweils die beiden Steuerspulen (711,721;731,741), die um denselben Statorzahn gewickelt sind, aufeinander gewickelt sind, sodass die äussere Steuerspule (711;731) die innere Steuerspule (721;741) umgibt.

13. Drehantrieb nach einem der vorangehenden Ansprüche mit zwei separaten Kontrollsystemen (5a,5b), von denen jedes die folgenden Komponenten umfasst:
- mindestens zwei Positionssensoren (81,82,83,84) zur Erfassung der radialen Position des Rotors (3) im Stator (2);
- Mittel (91,92) zur Bestimmung des Rotorwinkels;
- mindestens drei Leistungsverstärker (41) zur Versorgung der einzelnen Stränge der Antriebs- (6) und der Steuerwicklung (7);
- eine Signalverarbeitungs- und Regeleinrichtung (50a,50b) zur Regelung des Antriebs und der Position des Rotors (3) sowie zur Ansteuerung der Leistungsverstärker (41); wobei jedes Kontrollsystem (5a,5b) mindestens eine Antriebsphase und mindestens zwei Steuerphasen ansteuert.

14. Drehantrieb nach Anspruch 13, bei welchem Kommunikationsmittel (58,59,59') zur Kommunikation zwischen den beiden Kontrollsystemen (5a,5b) vorgesehen sind.

15. Drehantrieb nach Anspruch 14, bei welchem die beiden Kontrollsysteme (5a,5b) über die Kommunikationsmittel (58,59,59') Werte für die Position des Rotors, die jeweils mit Hilfe der Positionssensoren (81-84) ermittelt werden, und/oder Werte für den Rotorwinkel, die jeweils mit Hilfe der Mittel (91,92) zur Bestimmung des Rotorwinkels (91,92) ermittelt werden, austauschen, die Werte für die Position und/oder den Rotorwinkel auf Fehler überprüfen, und gegebenenfalls fehlerkorrigierte Werte bestimmen, welche dann beide Kontrollsysteme (5a,5b) zur Regelung der radialen Lage des Rotors (3) beziehungsweise zur Antriebsregelung verwenden.

16. Drehantrieb nach einem der Ansprüche 13-15, bei welchem Fehlererkennungsmittel (203,414,57a,57b,52a,52b) vorgesehen sind, mit denen Fehler in den Kontrollsystemen (5a,5b) und/oder in den einzelnen Antriebsphasen und/oder in den einzelnen Steuerphasen und/oder in der Energieversorgung detektierbar sind.

17. Drehantrieb nach Anspruch 16, bei welchem die Fehlererkennungsmittel im Falle der Detektion eines Fehlers in Abhängigkeit von der Art des Fehlers diesen beheben oder den Drehantrieb in einen geeigneten von mehreren möglichen Fehlermodi umschalten.

18. Drehantrieb nach einem der vorangehenden Ansprüche, bei welchem jedes Kontrollsystem (5a,5b) zumindest teilweise in Form von Elektronikprints ausgestaltet ist, die im Innern des Drehantriebs (1;10) angeordnet sind.

19. Drehantrieb nach Anspruch 18, bei welchem die im Innem des Drehantriebs angeordneten Elektronikprints alle Leistungsverstärker (41) umfassen und so angeordnet sind, dass die einzelnen Leistungsverstärker (41) jeweils unmittelbar mit den zugehörigen Antriebs- bzw. Steuerspulen verbunden sind.

20. Drehantrieb nach einem der vorangehenden Ansprüche ausgestaltet als Tempelmotor, bei welchem ein Auswertemodul (8) für die Positionssensoren (81-84) vorgesehen ist, welches als Elektronikprint ausgestaltet ist und derart auf den kürzeren Schenkeln (SK) der Statorzähne (21-28) angeordnet ist, dass sich die auf dem Elektronikprint vorgesehenen Bauteile in dem freien Raum zwischen den Statorzähnen (21-28) befinden.

21. Blutpumpe mit einem elektrischen Drehantrieb gemäss einem der vorangehenden Ansprüche, wobei der Rotor (3) des Drehantriebs permanentmagnetisch erregt ist und mehrere Flügel (34) zum Fördern des Bluts aufweist, sodass der Rotor (3) des Drehantriebs auch als Pumpenrotor dient.

## Claims

1. Fault-tolerant electrical rotary drive, designed as a bearingless motor, having a magnetically journalled rotor (3) and a stator (2) which includes a drive winding (6) which has at least two strands (61, 62) for producing a magnetic drive field which produces a torque on the rotor (3), and a control winding (7) having at least three strands (71, 72, 73, 74) for producing a magnetic control field with which the position of the rotor (3) with respect to the stator (2) can be regulated, with each strand (61, 62) of the drive winding (6) belonging to a different electrical drive phase and with each strand (71 - 74) of the control winding (7) belonging to a different electrical control phase, and having a setting device (4) which supplies each strand (61, 62) of the drive winding (6) and each strand (71 - 74) of the control winding (7) in each case with a phase current or a phase voltage as a setting parameter, **characterized in that** a control apparatus is provided which allows an independent control of each single drive phase and of each single control phase so that the setting parameter for each strand (61, 62) of the drive winding (6) and for each strand (71 - 74) of the control winding (7) can be regulated using the setting device (4) independently of the setting parameters for the other strands, with the setting device (4) including a separate bipolar amplifier (41) for each strand (61, 62) of the drive winding (6) and for each strand (71 - 74) of the control winding (7).

2. Rotary drive in accordance with claim 1, in which the drive winding (6) has a number of pole pairs p and the control winding (7) has a number of pole pairs p±1.

3. Rotary drive in accordance with any one of the preceding claims, designed as a temple motor, in which the stator (2) has a plurality of stator teeth (21 - 28) which are connected by a yoke (20) and which are formed in each case in L shape, with the longer limb (SL) extending in the axial direction which is defined by the desired axis of rotation (A) of the rotor (3), and with the shorter limb (SK) extending radially inwardly.

4. Rotary drive in accordance with any one of the preceding claims, having exactly three drive phases and having exactly three control phases.

5. Rotary drive in accordance with any one of the claims 1 - 4, having exactly two drive phases and having exactly four control phases.

6. Rotary drive in accordance with any one of the preceding claims, in which the stator has exactly eight stator teeth (21 - 28), between which the rotor (3) is journalled.

7. Rotary drive in accordance with claim 6, having two drive phases, with the drive winding (6) including four concentrated drive coils (611, 621), and with each drive coil (611, 621) being wound around two directly adjacent stator teeth (21 - 28), with in each case two diametrically oppositely lying drive coils (611; 621) forming a strand (61; 62) of the drive winding (6) and thus belonging to the same drive phase.

8. Rotary drive in accordance with any one of the preceding claims, in which the control winding (7) includes a plurality of concentrated control coils (711, 721, 731, 741), each of which is wound around a different stator tooth.

9. Rotary drive in accordance with any one of the preceding claims, in which the control winding (7) includes a plurality of concentrated control coils (711, 721, 731, 741), with the control coil which is wound around the first stator tooth and the control coil which is wound around the next but one stator tooth when viewed in the peripheral direction belonging in each case to the same control phase.

10. Rotary drive in accordance with any one of the preceding claims, having four control phases, in which the control winding (7) includes eight concentrated control coils (711, 721, 731, 741), with in each case two control coils (711; 721; 731; 741) forming a strand (71; 72; 73; 74) of the control winding (7) and thus belonging to the same control phase.

11. Rotary drive in accordance with any one of the preceding claims, having four control phases, in which the control winding (7) includes sixteen concentrated control coils (711, 721, 731, 741), with two control coils (711, 721, 731, 741) in each case being provided on each stator tooth, and with in each case four control coils (711; 721; 731; 741), which are wound around four different stator teeth, being connected together to form a strand (71; 72; 73; 74) of the control winding (7) such that when viewed in the peripheral direction the control coils which are wound around directly adjacent stator teeth belong in each case to different strands of the control winding.

12. Rotary drive in accordance with claim 11, in which the two control coils (711, 721; 731, 741) which are wound around the same stator tooth are in each case wound one on the other, so that the outer control coil (711; 731) surrounds the inner control coil (721; 741).

13. Rotary drive in accordance with any one of the preceding claims, having two separate control systems (5a, 5b), each of which includes the following components:
- at least two position sensors (81, 82, 83, 84) for detecting the radial position of the rotor (3) in the stator (2);
- means (91, 92) for determining the rotor angle,
- at least three power amplifiers (41) for supplying the individual strands of the drive winding (6) and of the control winding (7),
- a signal processing and regulation device (50a, 50b) for the regulation of the drive and the position of the rotor (3) as well as for controlling the power amplifiers (41),
with each control system (5a, 5b) controlling at least one drive phase and at least two control phases.

14. Rotary drive in accordance with claim 13, in which communication means (58, 59, 59') are provided for communication between the two control systems (5a, 5b).

15. Rotary drive in accordance with claim 14, in which the two control systems (5a, 5b) exchange via the communication means (58, 59, 59') values for the position of the rotor, which are in each case determined with the help of position sensors (81 - 84), and/or values for the rotor angle, which are in each case determined with the help of the means (91, 92) for determining the rotor angle (91, 92), check the values for the position and/or the rotor angle for errors, and where appropriate determine fault-corrected values, which both control systems (5a, 5b) then use for the regulation of the radial position of the rotor (3) or for the drive regulation respectively.

16. Rotary drive in accordance with any one of the claims 13 - 15, in which fault detection means (203, 414, 57a, 57b, 52a, 52b) are provided, with which faults can be detected in the control systems (5a, 5b) and/or in the individual drive phases and/or in the individual control phases and/or in the energy supply.

17. Rotary drive in accordance with claim 16, in which in the event of the detection of a fault the fault detection means eliminate it or switch over the rotary drive into a suitable fault mode out of a plurality of possible ones in dependence on the nature of the fault.

18. Rotary drive in accordance with any one of the preceding claims, in which each control system (5a, 5b) is designed at least partly in the form of electronic prints which are arranged in the interior of the rotary drive (1; 10).

19. Rotary drive in accordance with claim 18, in which the electronic prints which are arranged in the interior of the rotary drive include all power amplifiers (41) and are arranged such that the individual power amplifiers (41) are in each case directly connected to the associated drive coils and control coils respectively.

20. Rotary drive in accordance with any one of the preceding claims, designed as a temple motor, in which an evaluation module (8) is provided for the position sensors (81 - 84) which is designed as an electronic print and which is arranged on the shorter limbs (SK) of the stator teeth (21 - 28) such that the components which are provided on the electronic print are located in the free space between the stator teeth (21 - 28).

21. Blood pump having an electrical rotary drive in accordance with any one of the preceding claims, wherein the rotor (3) of the rotary drive is permanent-magnetically excited and has a plurality of vanes (34) for the forwarding of the blood, so that the rotor (3) of the rotary drive also serves as pump rotor.

## Revendications

1. Entraînement électrique rotatif tolérant aux erreurs, réalisé comme moteur sans palier, avec un rotor (3) logé magnétiquement et un stator (2) qui comprend un enroulement d'entraînement (6) présentant au moins deux conducteurs (61, 62) pour la production d'un champ d'entraînement magnétique, qui provoque un couple de rotation sur le rotor (3), et un enroulement de commande (7) présentant au moins trois conducteurs (71, 72, 73, 74) pour la production d'un champ de commande magnétique au moyen duquel la position du rotor (3) par rapport au stator (2) est réglable, où chaque conducteur (61, 62) de l'enroulement d'entraînement (6) appartient à une autre phase d'entraînement électrique, et chaque conducteur (71-74) de l'enroulement de commande (7) à une autre phase de commande électrique, et avec une installation de réglage (4) qui alimente chaque conducteur (61, 62) de l'enroulement d'entraînement (6) et chaque conducteur (71-74) de l'enroulement de commande (7) respectivement en courant de phase ou en tension de phase, comme grandeur de réglage, **caractérisé en ce qu'**un dispositif de contrôle (5) est prévu qui permet une commande indépendante de chaque phase d'entraînement individuelle et de chaque phase de commande individuelle, de sorte qu'au moyen de l'installation de réglage (4), la grandeur de réglage pour chaque conducteur (61, 62) de l'enroulement d'entraînement (6) et pour chaque conducteur (71-74) de l'enroulement de commande (7) est réglable indépendamment des grandeurs de réglage des autres conducteurs, où l'installation de réglage (4) comprend pour chaque conducteur (61,62) de l'enroulement d'entraînement (6) et pour chaque conducteur (71-74) de l'enroulement de commande (7) un amplificateur de puissance bipolaire séparé (41).

2. Entraînement électrique rotatif selon la revendication 1, dans lequel l'enroulement d'entraînement (6) présente un nombre de paires polaires p et l'enroulement de commande (7) un nombre de paires polaires p±1.

3. Entraînement électrique rotatif selon l'une des revendications précédentes, réalisé comme moteur temple, dans lequel le stator (2) présente plusieurs dents de stator (21-28) reliées par un shunt magnétique (20), qui sont réalisées chacune en forme de L, où la branche plus longue (SL) s'étend dans la direction axiale qui est fixée par l'axe de rotation de consigne (A) du rotor (3), et la branche plus courte (SK) s'étend radialement vers l'intérieur.

4. Entraînement électrique rotatif selon l'une des revendications précédentes, avec précisément trois phases d'entraînement et avec précisément trois phases de commande.

5. Entraînement électrique rotatif selon l'une des revendications 1 à 4, avec précisément deux phases d'entraînement et avec précisément quatre phases de commande.

6. Entraînement électrique rotatif selon l'une des revendications précédentes, dans lequel le stator présente précisément huit dents de stator (21-28) entre lesquels est logé le rotor (3).

7. Entraînement électrique rotatif selon la revendication 6, avec deux phases d'entraînement, où l'enroulement d'entraînement (6) comprend quatre bobines d'enroulement concentrées (611,621), et chaque bobine d'entraînement (611,621) est enroulée autour de deux dents de stator directement avoisinantes (21-28), où respectivement deux bobines d'entraînement (611;621) diamétralement opposées forment un conducteur (61;62) de l'enroulement d'entraînement (6) et appartiennent donc à la même phase d'entraînement.

8. Entraînement électrique rotatif selon l'une quelconque des revendications précédentes, dans lequel l'enroulement de commande (7) comprend plusieurs bobines de commande concentrées (711,721,731,741) dont chacune est enroulée autour d'une autre dent de stator.

9. Entraînement électrique rotatif selon l'une des revendications précédentes, dans lequel l'enroulement de commande (7) comprend plusieurs bobines de commande concentrées (711,721,731,741), où à chaque fois la bobine de commande enroulée autour d'une première dent de stator et la bobine de commande enroulée, vue dans la direction périphérique, autour de la surprochaine dent de stator font partie de la même phase de commande.

10. Entraînement électrique rotatif selon l'une des revendications précédentes avec quatre phases de commande, dans lequel l'enroulement de commande (7) comprend huit bobines de commande concentrées (711,721,731,741), où à chaque fois deux bobines de commande (711,721,731,741) forment un conducteur (71,72,73,74) de l'enroulement de commande (7) et font donc partie de la même phase de commande.

11. Entraînement électrique rotatif selon l'une des revendications précédentes avec quatre phases de commande, dans lequel l'enroulement de commande (7) comprend seize bobines de commande concentrées (711,721,731,741), où sont prévues sur chaque dent de stator à chaque fois deux bobines de commande (711,712,731,741), et où à chaque fois quatre bobines de commande (711;721;731;741) qui sont enroulées autour de quatre dents de stator différentes, sont commutées de telle sorte en un conducteur (71;72;73;74) de l'enroulement de commande (7) que, vues dans la direction périphérique, à chaque fois les bobines de commande enroulées autour des dents de stator directement avoisinantes appartiennent à des conducteurs différents de l'enroulement de commande.

12. Entraînement électrique rotatif selon la revendication 11, dans lequel à chaque fois les deux bobines de commande (711,721;731,741), qui sont enroulées autour de la même dent de stator, sont enroulées l'une sur l'autre de sorte que la bobine de commande extérieure (711;731) entoure la bobine de commande intérieure (721;741).

13. Entraînement électrique rotatif selon l'une des revendications précédentes, avec deux systèmes de contrôle séparés (5a,5b), dont chacun comprend les composants suivants:
- au moins deux capteurs de position (81,82,83,84) pour la détection de la position radiale du rotor (3) dans le stator (2);
- des moyens (91,92) pour la détermination de l'angle du rotor;
- au moins trois amplificateurs de puissance (41) pour l'alimentation des différents conducteurs de l'enroulement d'entraînement (6) et de commande (7);
- une installation de traitement de signaux et de régulation (50a,50b) pour la régulation de l'entraînement et de la position du rotor (3) ainsi que pour la commande de l'amplificateur de puissance (41); où chaque système de contrôle (5a,5b) commande au moins une phase d'entraînement et au moins deux phases de commande.

14. Entraînement électrique rotatif selon la revendication 13, dans lequel sont prévus des moyens de communication (58,59,59') pour la communication entre les deux systèmes de contrôle (5a,5b).

15. Entraînement électrique rotatif selon la revendication 14, dans lequel les deux systèmes de contrôle (5a,5b) échangent par les moyens de communication (58,59,59') des valeurs pour la position du rotor, qui sont déterminées à chaque fois à l'aide des capteurs de position (81-84) et/ou des valeurs pour l'angle du rotor qui sont déterminées à chaque fois à l'aide des moyens (91,92) pour la détermination de l'angle du rotor (91,92), surveillent les valeurs pour la position et/ou l'angle du rotor quant à des erreurs et définissent le cas échéant des valeurs dont les erreurs sont corrigées, qui sont utilisées ensuite par les deux systèmes de contrôle (5a,5b) pour la régulation de la position radiale du rotor (3) respectivement pour la régulation de l'entraînement.

16. Entraînement électrique rotatif selon l'une des revendications 13 à 15, dans lequel sont prévus des moyens de détection d'erreur (203,414,57a,57b,52a,52b) au moyen desquels peuvent être détectées des erreurs dans les systèmes de contrôle (5a,5b) et/ou dans les phases d'entraînement individuelles et/ou dans les phases de commande individuelles et/ou dans l'alimentation en énergie.

17. Entraînement électrique rotatif selon la revendication 16, dans lequel les moyens de détection d'erreur, dans le cas de la détection d'une erreur, en fonction du type de l'erreur, corrigent celle-ci ou commutent l'entraînement électrique rotatif en un mode approprié de plusieurs modes d'erreur possibles.

18. Entraînement électrique rotatif selon l'une des revendications précédentes, dans lequel chaque système de contrôle (5a,5b) est réalisé au moins partiellement sous forme d'impressions électroniques qui sont disposées dans l'intérieur de l'entraînement électrique rotatif (1;10).

19. Entraînement électrique rotatif selon la revendication 18, dans lequel les impressions électroniques disposées dans l'intérieur de l'entraînement électrique rotatif comprennent tous les amplificateurs de puissance (41) et sont disposées de telle sorte que les amplificateurs de puissance individuels (41) sont reliés à chaque fois directement aux bobines d'entraînement respectivement de commande associées.

20. Entraînement électrique rotatif selon l'une des revendications précédentes, réalisé comme moteur temple, dans lequel est prévu un module d'évaluation (8) pour les capteurs de position (81-84), qui est réalisé comme impression électronique et est disposé de telle sorte sur les branches plus courtes (SK) des dents de stator (21-28) que les composants prévus sur l'impression électronique se trouvent dans l'espace libre entre les dents de stator (21-28).

21. Pompe de sang avec un entraînement électrique rotatif selon l'une des revendications précédentes, où le rotor (3) de l'entraînement électrique rotatif est excité par aimant permanent et présente plusieurs ailettes (34) pour le convoyage du sang de sorte que le rotor (3) de l'entraînement électrique rotatif sert aussi de rotor de pompe.
